# EUROPEAN PATENT SPECIFICATION

(11) **EP 1 572 961 B1**
(45) Date of publication and mention of the grant of the patent: **18.11.2009**
(21) Application number: 03773145.2
(22) Date of filing: 24.10.2003
(51) Int. Cl.: A01N 37/18, A61K 38/00, C07K 1/00, C07K 14/00, C07K 17/00

(54) **ACTRIIB FUSION POLYPEPTIDES AND USES THEREFOR**
ACTRIIB-FUSIONSPOLYPEPTIDE UND VERWENDUNGEN DAFÜR
POLYPEPTIDES DE FUSION ACTRIIB ET UTILISATIONS ASSOCIEES

(30) Priority: 25.10.2002 US 421041 P
(43) Date of publication of application: 14.09.2005
(73) Proprietor: Wyeth, Madison, NJ 07940 (US)
(72) Inventor: WOLFMAN, Neil, M., Andover, MA 02030 (US); BOUXSEIN, Mary, L., Brookline, MA 02446 (US)
(74) Representative: Schlich, George William
(86) International application number: PCT/US2003/031516
(87) International publication number: WO 2004/039948

(56) References cited:
- WO-A-02/10214
- WO-A-2005/028517
- WO-A2-02/068650
- WO-A2-2004/108157
- US-A- 5 545 616
- US-A- 5 573 924
- US-A- 6 093 547
- US-A- 6 162 896
- LEE S-J ET AL: "Regulation of myostatin activity and muscle growth" PROCEEDINGS OF THE NATIONAL ACADEMY OF SCIENCES OF USA, NATIONAL ACADEMY OF SCIENCE, WASHINGTON, DC, US, vol. 98, no. 16, 31 July 2001 (2001-07-31), pages 9306-9311, XP002281005 ISSN: 0027-8424
- KOMESLI S ET AL: "Chimeric extracellular domain of type II transforming growth factor (TGF)-beta receptor fused to the Fc region of human immunoglobulin as a TGF-beta antagonist" EUROPEAN JOURNAL OF BIOCHEMISTRY, BERLIN, DE, vol. 254, no. 3, 15 June 1998 (1998-06-15), pages 505-513, XP000856458 ISSN: 0014-2956
- LEE S-J ET AL: "Regulation of muscle growth by multiple ligands signaling through activin type II receptors" PROCEEDINGS OF THE NATIONAL ACADEMY OF SCIENCES OF USA, NATIONAL ACADEMY OF SCIENCE, WASHINGTON, DC, US, vol. 102, no. 50, 13 December 2005 (2005-12-13), pages 18117-18122, XP002428860 ISSN: 0027-8424
- GARG R. ET AL: 'Cloning of zebrafish activin type IIB receptor (ActRIIB) cDNA and mRNA expression of ActRIIB in embryos and adult tissues' MOLECULAR AND CELLULAR ENDOCRINOLOGY vol. 153, 1999, pages 169 - 181

## Description

### TECHNICAL FIELD

This technical field relates to inhibitors of growth and differentiation factor-8 (GDF-8), including soluble forms of activin type II receptors, and fragments thereof, especially those that inhibit GDF-8 activity *in vivo.* The field further relates to methods for preventing, or treating degenerative disorders of bone.

### BACKGROUND

The TGF-β family is a number of structurally-related growth factors, all of which possess physiologically important growth-regulatory and morphogenetic properties (Kingsley et al. (1994) Genes Dev., 8:133-146; Hoodless et al. (1998) Curr. Topics Microbiol. Immunol., 228:235-272). These factors include bone morphogenetic proteins (BMP), activin, inhibin, mullerian inhibiting substance, glial-derived neurotrophic factor, and a still growing number of growth and differentiation factors (GDF), such as GDF-8. Many of these proteins are highly homologous. For example, human BMP-11, also known as GDF-11, is 90% identical to GDF-8 at the amino-acid level (Gamer et al. (1999) Dev. Biol. 208:222-232; http://www.ronmyrick.comNakashima et al. (1999) Mech. Dev. 80:185-189).

Most members of the TGF-β family are known to transduce their signals through the formation of heteromeric complexes of two different types of serine/threonine kinase receptors expressed on the cell surface, *i.e*., type I receptors of about 50-55 kDa and type II receptors of more than 70 kDa. Type I receptors do not bind ligands directly; rather, they participate in signal transduction by associating with the type II receptors, which do bind ligand molecules. The TGF-β system is highly conserved throughout the animal kingdom. (For a review of the TGF-β system, see Massague (2000) Nature Rev. Mol. Cell Biol. 1:16-178; and Moustakas et al. (2001) J. Cell Sci. 114:4359-4369)

Activin type II receptor has been previously described in U.S. Patent No. 5,885,794. Activin was originally purified from ovarian follicular fluid as a protein that has a stimulatory effect on production of follicle-stimulating hormone in the pituitary gland. Five isoforms of activin type II receptor have been identified in activin-responsive cells. Based on *in vitro* studies, these receptors may be shared by members of the TGF-β family (Attisano et al. (1996) Mol. Cell. Biol. 16:1066-1073). The present invention is based, in part, on the discovery that the type II activin receptor, termed ActRIIB, can bind to growth and differentiation factor-8 (GDF-8) in addition to activin.

GDF-8 is involved in the regulation of critical biological processes in the skeletal muscle and osteogenesis. GDF-8 is highly expressed in the developing and adult skeletal muscle. GDF-8 knockout transgenic mice are characterized by a marked hypertrophy and hyperplasia of the skeletal muscle (McPherron et al. (1997) Nature 387:83-90) and altered cortical bone structure (Hamrick et al. (2000) Bone 27 (3):343-349). Similar increases in skeletal muscle mass are evident in naturally occurring mutations of GDF-8 in cattle (Ashmore et al. (1974) Growth 38:501-507; Swatland et al. (1994) J. Anim. Sci. 38:752-757; McPherron et al. (1997) Proc. Natl. Acad. Sci. U.S.A. 94:12457-12461; and Kambadur et al. (1997) Genome Res. 7:910-915). Studies have indicated that muscle wasting associated with HIV-infection is accompanied by an increase in GDF-8 expression (Gonzalez-Cadavid et al. (1998) Proc. Natl. Acad. Sci. U.S.A. 95:14938-14943). GDF-8 has also been implicated in the production of muscle-specific enzymes (*e.g*., creatine kinase) and proliferation of myoblast cells (WO 00/43781). In addition to its growth-regulatory and morphogenetic properties, GDF-8 may also be involved in a number of other physiological processes, including glucose homeostasis in the development of type 2 diabetes, impaired glucose tolerance, metabolic syndromes (*e.g*., syndrome X), insulin resistance induced by trauma such as burns or nitrogen imbalance, and adipose tissue disorders, such as obesity (Kim et al. (2001) BBRC 281:902-906).

A number of human and animal disorders are associated with functionally impaired muscle tissue, *e.g*., muscular dystrophy (including Duchenne's muscular dystrophy), amyotrophic lateral sclerosis (ALS), muscle atrophy, organ atrophy, frailty, congestive obstructive pulmonary disease, sarcopenia, cachexia, and muscle wasting syndrome caused by other diseases and conditions. To date, very few reliable or effective therapies have been developed to treat these disorders.

There are also a number of conditions associated with a loss of bone, which include osteoporosis and osteoarthritis, especially in the elderly and/or postmenopausal women. In addition, metabolic bone diseases and disorders include low bone mass due to chronic glucocorticoid therapy, premature gonadal failure, androgen suppression, vitamin D deficiency, secondary hyperparathyroidism, nutritional deficiencies, and anorexia nervosa. Currently available therapies for these conditions work by inhibiting bone resorption. A therapy that promotes new bone formation would be a desirable alternative to these therapies.

Thus, a need exists to develop new therapies that contribute to an overall increase of muscle mass and/or bone density, especially, in humans. It is an object of the present invention to provide safe and effective therapeutic methods for bone-associated disorders. It is another object of the invention to provide methods of increasing bone density in mammals. It is yet another object of the invention to provide inhibitors of GDF-8 that are safe and effective *in vivo.*

Still another object of the invention is to provide soluble forms of activin type II receptor ActRIIB and/or functional fragments thereof that are stable *in vivo* and bind GDF-8 with high specificity and affinity. WO 02/10214 describes a substantially purified growth differentiation factor (GDF) receptor, including a GDF-8 receptor for treatment of pathologic conditions related to muscle or adipose tissue.

Additional objects of the invention will be set forth in part in the description which follows, and in part will be obvious from the description or may be learned by practice of the invention.

Various objects, aspects and advantages of the invention will be realized and attained by means of the elements and combinations particularly pointed out in the appended claims.

### SUMMARY

Accordingly, the present invention provides use of an Activin type IIB receptor (ActRIIB) fusion polypeptide in manufacture of a medicament for treatment or prevention of a bone degenerative disorder in a mammal, wherein the ActRIIB fusion polypeptide comprises (a) an amino acid sequence that is at least 95% identical to amino acids 23 to 138 of SEQ ID NO:3 and is capable of binding to growth and differentiation factor-8 (GDF-8) and (b) the constant region of an IgG, IgA, IgE, or IgM antibody.

The invention also provides a fusion protein comprising the amino acid sequence of SEQ ID NO:3.

The invention further provides an isolated nucleic acid encoding the fusion protein of the invention, an expression vector, comprising the nucleic acid of the invention and a host cell comprising the vector of the invention.

The invention additionally provides a method for identifying inhibitors of GDF 8, comprising:
(a) preparing a first binding mixture comprising the ActRIIB fusion polypeptide of the invention and GDF 8;
(b) measuring the amount of binding between the ActRIIB fusion polypeptide and GDF 8 in the first mixture;
(c) preparing a second binding mixture comprising the ActRIIB fusion polypeptide, GDF 8 and a test compound; and
(d) measuring the amount of binding between the ActRIIB fusion polypeptide and GDF 8 in the second mixture.

Yet further provided by the invention is use of an ActRIIB fusion polypeptide in manufacture of a medicament for increasing trabecular bone density, wherein the ActRIIB fusion polypeptide comprises (a) an amino acid sequence that is at least 95% identical to amino acids 23 to 138 of SEQ ID NO:3 and is capable of binding to growth and differentiation factor-8 (GDF-8) and (b) the constant region of an IgG, IgA, IgE, or IgM antibody.

Methods for treating bone degenerative disorders are described herein. The methods are also useful for increasing bone density in normal animals.

Also described are methods for inhibiting GDF-8 activity associated with negative regulation of bone density.

Stabilized soluble ActRIIB forms and fragments thereof that bind and inhibit GDF-8 *in vitro* and *in vivo* are provided. The presently disclosed soluble ActRIIB forms possess pharmacokinetic properties that make them suitable as therapeutic agents.

Other aspects provide compositions containing the presently described ActRIIB fusion polypeptides and their use in methods of inhibiting or neutralizing GDF-8, including methods of treatment of the human or animals. The disclosed ActRIIB fusion polypeptides may be used to treat or prevent conditions in which an increase in bone density is desirable. Exemplary disease and disorders include bone degenerative disease such as osteoarthritis and osteoporosis. The modified ActRIIB forms utilized in the methods of the invention are thus ActRIIB fusion polypeptides comprising (a) a first amino acid sequence derived from the ActRIIB extracellular domain and (b) a second amino acid sequence derived from the constant region of an antibody.

The first sequence may comprise all or a portion of an extracellular domain of human ActRIIB, or may be a mutation of such a sequence. The second sequence may be derived from the Fc portion of an antibody, or may be a mutation of such a sequence.

The second sequence can be linked to the C-terminus or the N-terminus of the first amino acid sequence, with or without being linked by a linker sequence.

Therapeutic methods for treating bone degenerative disorders are also described. Exemplary disease and disorders include bone degenerative disease such as osteoporosis.

In addition, the presently disclosed ActRIIB fusion polypeptides may be used as a diagnostic tool to quantitatively or qualitatively detect GDF-8 or fragments thereof in a biological sample. The presence or amount of GDF-8 detected can be correlated with one or more of the medical conditions listed above.

An isolated nucleic acid encoding an ActRIIB fusion polypeptide used in the methods of the invention is also provided. Further provided are expression vectors comprising the nucleic acid; host cells comprising the expression vectors; and methods for producing the nucleic acid.

Yet another aspect provides a method for identifying therapeutic agents useful in treatment of muscle and bone disorders.

It is to be understood that both the foregoing general description and the following detailed description are exemplary and explanatory only and are not restrictive of the invention, as claimed.

### BRIEF DESCRIPTION OF THE FIGURES

Figure 1 shows binding of biotinylated GDF-8 and BMP-11 to ActRIIB-Fc.

Figure 2 shows results of reporter gene assays in which ActRIIB-Fc has been tested.

Figure 3 depicts results of a pharmacokinetic study in which C57B6/SCID mice utilizing a single intravenous (IV) or intraperitoneal (IP) administration of ActRIIB-Fc.

### BRIEF DESCRIPTION OF THE SEQUENCES

The following table is provided as a reference for the sequences referred to in this application.

| **Reference** | **Type*** | **Sequence** |
|---|---|---|
| SEQ ID NO:1 | AA | ActRIIB |
| SEQ ID NO:2 | AA | GDF-8 |
| SEQ ID NO:3 | AA | ActRIIB-Fc |
| SEQ ID NO:4 | DNA | Encodes SEQ ID NO:3 |
| SEQ ID NO:5 | AA | Linker |
| SEQ ID NO:6 | AA | Enterokinase cleavage site |

| | | |
|---|---|---|
| *AA = amino acid | | |

### DETAILED DESCRIPTION

### 1. Definitions

The term "ActRIIB" refers to any isoform of activin type II receptor or a fragment thereof that is capable of specifically binding GDF-8. The term is not limited to any particular species of origin, method of production, and other characteristics of ActRIIB. The term includes recombinantly produced ActRIIB or its fragments, and particularly, the GDF-8 binding domain of human ActRIIB. The term also encompasses allelic and splice variants of ActRIIB, their homologues, and orthologues and sequences thereof containing introduced mutations (substitutions, additions, or deletions), *e.g*., those introduced by recombinant techniques.

The term "degenerative disorder of muscle, bone, or glucose homeostasis" refers to a number of disorders and diseases associated with GDF-8 and/or other members of the TGF-β superfamily, *e.g*., BMP-11. Example of such disorders include, but are not limited to, metabolic disorders such as type 2 diabetes, impaired glucose tolerance, metabolic syndrome (*e.g*., syndrome X), and insulin resistance induced by trauma (*e.g*., burns or nitrogen imbalance); adipose tissue disorders (*e.g*., obesity); muscle and neuromuscular disorders such as muscular dystrophy (including Duchenne's muscular dystrophy); amyotrophic lateral sclerosis (ALS); muscle atrophy; organ atrophy; frailty; carpal tunnel syndrome; congestive obstructive pulmonary disease; and sarcopenia, cachexia and other muscle wasting syndromes. Other examples include osteoporosis, especially in the elderly and/or postmenopausal women; glucocorticoid-induced osteoporosis; osteopenia; osteoarthritis; and osteoporosis-related fractures. Yet further examples include low bone mass due to chronic glucocorticoid therapy, premature gonadal failure, androgen suppression, vitamin D deficiency, secondary hyperparathyroidism, nutritional deficiencies, and anorexia nervosa.

The term "effective amount" refers to that amount of the compound which results in amelioration of symptoms in a patient or a desired biological outcome (*e.g*., increasing skeletal muscle mass and/or bone density). Such amount should be sufficient to reduce the activity of GDF-8 associated with negative regulation of skeletal muscle mass and bone density. The effective amount can be determined as described in the subsequent sections.

The term "GDF-8 binding domain," when used in relation to ActRIIB, refers to the extracellular domain of ActRIIB or a part thereof necessary for binding to GDF-8, *i.e*., a portion of the ActRIIB extracellular domain responsible for specific binding to GDF-8.

The term "TGF-β superfamily" refers to a family of structurally related growth factors. This family of related growth factors is well known in the art (Kingsley et al. (1994) Genes Dev. 8:133-146; Hoodless et al. (1998) Curr. Topics Microbiol. Immunol. 228:235-72). The TGF-β superfamily includes bone morphogenetic proteins (BMP), activin, inhibin, mullerian inhibiting substance, glial-derived neurotrophic factor, and a still growing number of growth and differentiation factors (GDF), such as GDF-8 (myostatin). Many of such proteins are structurally and/or functionally related to GDF-8. For example, human BMP-11, also known as GDF-11, is 90% identical to GDF-8 at the amino-acid level (Gamer et al. (1999) Dev. Biol. 208:222-232; Nakashima et al. (1999) Mech. Dev. 80:185-189).

The term "GDF-8" refers to a specific growth and differentiation factor-8. The term refers to the full-length unprocessed precursor form of GDF-8, as well as the mature and propeptide polypeptides resulting from post-translational cleavage. The term also refers to any fragments and variants of GDF-8 that retain one or more biological activities associated with GDF-8 as discussed herein. The amino acid sequence of mature human GDF-8 is provided in SEQ ID NO:2. The present invention relates to GDF-8 from all vertebrate species, including, but not limited to, human, bovine, chicken, murine, rat, porcine, ovine, turkey, baboon, and fish (for sequence information, see, *e.g*., McPherron et al. (1997) Proc. Natl. Acad. Sci. U.S.A. 94:12457-12461).

The term "mature GDF-8" refers to the protein that is cleaved from the carboxy-terminal domain of the GDF-8 precursor protein. The mature GDF-8 may be present as a monomer, homodimer, or in a GDF-8 latent complex. Depending on conditions, mature GDF-8 may establish equilibrium between any or all of these different polypeptides. In its biologically active form, the mature GDF-8 is also referred to as "active GDF-8."

The term "GDF-8 propeptide" refers to the polypeptide that is cleaved from the amino-terminal domain of the GDF-8 precursor protein. The GDF-8 propeptide is capable of binding to the propeptide binding domain on the mature GDF-8.

The term "GDF-8 latent complex" refers to the complex of proteins formed between the mature GDF-8 homodimer and the GDF-8 propeptide. It is believed that two GDF-8 propeptides associate with the two molecules of mature GDF-8 in the homodimer to form an inactive tetrameric complex. The latent complex may include other GDF-8 inhibitors in place of or in addition to one or both of the GDF-8 propeptides.

The term "GDF-8 activity" refers to one or more of physiologically growth-regulatory or morphogenetic activities associated with active GDF-8 protein. For example, active GDF-8 is a negative regulator of skeletal muscle. Active GDF-8 can also modulate the production of muscle-specific enzymes (*e.g*., creatine kinase), stimulate myoblast proliferation, and modulate preadipocyte differentiation to adipocytes. Procedures for assessing GDF-8 activity *in vivo* and *in vitro* include, but are not limited to, for example, reporter gene assays (see Example 6) or *in vivo* tests involving measurements of muscle and/or bone parameters (see Examples 8, 9, and 10).

The term "Fc portion" refers to the C-terminal fragment of an immunoglobulin generated by proteolysis with papain, or a functional equivalent derived therefrom. The term "Fc portion" should be understood to encompass recombinantly produced Fc fragments, including those derived from any antibody isotype, *e.g*., IgG, IgA, IgE, IgM, and any of the isotype subclasses. The term "constant region of an antibody" refers to a C-terminal portion of an immunoglobulin, comprising the Fc portion and adjacent sequences so long as these sequences do not include variable regions of the antibody, such as complementarity determining regions (CDRs). The constant region of an antibody is the same in all antibodies of a particular isotype.

As used herein, the term "hybridization under stringent conditions" is intended to describe conditions for hybridization and washes under which nucleotide sequences that are significantly identical or homologous to each other remain complementarily bound to each other. The conditions are such that sequences at least about 70%, more preferably at least about 80%, even more preferably at least about 85-90% identical remain bound to each other. The percent identity is determined as described in Altschul et al. (1997) Nucleic Acids Res. 25:3389-3402.

Stringent conditions are known in the art and can be found in Current Protocols in Molecular Biology, John Wiley & Sons, Inc. (eds. Ausubel et al. 1995), sections 2, 4, and 6. Additionally, stringent conditions are described in Sambrook *et al.* (1989) Molecular Cloning: A Laboratory Manual, 2nd ed. Cold Spring Harbor Press, chapters 7, 9, and 11. An example of stringent hybridization conditions is hybridization in 4X sodium chloride/sodium citrate (SSC) at about 65-70°C or hybridization in 4X SSC plus 50% formamide at about 42-50°C, followed by one or more washes in 1X SSC, at about 65-70°C. When using nylon membranes, for instance, an additional non-limiting example of stringent hybridization conditions is hybridization in 0.25-0.5 M NaH₂PO₄, 7% SDS at about 65°C, followed by one or more washes at 0.02 M NaH₂PO₄, 1% SDS at 65°C. See, *e.g*., Church et al. (1984) Proc. Natl. Acad. Sci. U.S.A. 81:1991-1995. It will be understood that additional reagents may be added to hybridization and/or wash buffers, *e.g*., blocking agents (BSA or salmon sperm DNA), detergents (SDS), chelating agents (EDTA), Ficoll, PVP, etc.

The term "inhibitor," when used in relationship to GDF-8 or its activity, includes any agent capable of inhibiting activity, expression, processing, or secretion of GDF-8. Such inhibitors include proteins, antibodies, peptides, peptidomimetics, ribozymes, anti-sense oligonucleotides, double-stranded RNA, and other small molecules, which inhibit GDF-8. Such inhibitors are said to "inhibit," "neutralize," or "reduce" the biological activity of GDF-8 protein.

The terms "neutralize," "neutralizing," "inhibitory," and their cognates refer to a reduction in the activity of GDF-8 by a GDF-8 inhibitor, relative to the activity of GDF-8 in the absence of the same inhibitor. The reduction in activity is preferably at least about 10%, 20%, 30%, 40%, 50%, 60%, 70%, 80%, 90%, or higher.

The term "isolated" refers to a molecule that is substantially free of its natural environment. For instance, an isolated protein is substantially free of cellular material or other proteins from the cell or tissue source from which it is derived. The term refers to preparations where the isolated protein is sufficiently pure to be administered as a therapeutic composition or at least 70% to 80% (w/w) pure, at least 80%-90% pure, 90-95% pure; or at least 95%, 96%, 97%, 98%, 99%, or 100% pure.

The term "mammal" refers to any animal classified as such, including humans, domestic and farm animals, zoo, sports, or pet animals, such as dogs, horses, cats, sheep, pigs, cows, etc.

The term "specific interaction," or "specifically binds," or the like, means that two molecules form a complex that is relatively stable under physiologic conditions. The term is also applicable where, *e.g*., an antigen-binding domain is specific for a particular epitope, which is carried by a number of antigens, in which case the antibody carrying the antigen-binding domain will be able to bind to the various antigens carrying the epitope. Thus, an antibody may specifically bind, for example, BMP-11 and GDF-8 as long as it binds to the epitope, which is carried by both.

Specific binding is characterized by a high affinity and a low to moderate capacity. Nonspecific binding usually has a low affinity with a moderate to high capacity. Typically, the binding is considered specific when the affinity constant Kₐ is higher than 10⁶ M⁻¹, or preferably higher than 10⁸ M⁻¹. If necessary, nonspecific binding can be reduced without substantially affecting specific binding by varying the binding conditions. Such conditions are known in the art, and a skilled artisan using routine techniques can select appropriate conditions. The conditions are usually defined in terms of concentration of the ActRIIB fusion polypeptide, ionic strength of the solution, temperature, time allowed for binding, concentration of non-related molecules (*e.g*., serum albumin, milk casein), etc. Exemplary conditions are set forth in Examples 5 and 6

The phrase "substantially as set out" means that a relevant amino acid sequence is at least 70%, 75%, 80%, 85%, 90%, 95%, 97%, 98%, or 99% identical to a given sequence. By way of example, such sequences may be variants derived from various species, or they may be derived from the given sequence by truncation, deletion, amino acid substitution or addition. Percent identity between two amino acid sequences is determined by standard alignment algorithms such as, for example, Basic Local Alignment Tool (BLAST) described in Altschul et al. (1990) J. Mol. Biol. 215:403-410, the algorithm of Needleman et al. (1970) J. Mol. Biol 48:444-453, or the algorithm of Meyers et al. (1988) Comput. Appl. Biosci. 4:11-17.

The term "treatment" refers to both therapeutic treatment and prophylactic/preventative treatment. Those in need of treatment may include individuals already having a particular medical disorder as well as those who may ultimately acquire the disorder (*i.e*., those needing preventative measures, such as, for example, post-menopausal women with a family history of osteoporosis, or obese patients with a family history of type 2 diabetes or somewhat elevated blood sugar readings).

### II. ActRIIB Fusion Polypeptides

Described herein is a modified activin type II receptor ActRIIB that binds GDF-8 and inhibits its activity *in vitro* and/or *in vivo.* In particular, the presently disclosed ActRIIB fusion polypeptides inhibit the GDF-8 activity associated with negative regulation of skeletal muscle mass and bone density. The ActRIIB fusion polypeptides described herein are soluble and possess pharmacokinetic properties that make them suitable for therapeutic use, *e.g*., extended circulatory half-life and/or improved protection from proteolytic degradation.

The ActRIIB fusion polypeptides described herein comprise (a) a first amino acid sequence derived from the extracellular domain of ActRIIB and (b) a second amino acid sequence derived from the constant region of an antibody. The full amino acid and DNA sequences of a particular illustrative embodiment of the ActRIIB fusion protein are set forth in SEQ ID NO:3 and SEQ ID NO:4, respectively.

The first amino acid sequence is derived from all or a portion of the ActRIIB extracellular domain and is capable of binding GDF-8 specifically. Such a portion of the ActRIIB extracellular domain may also bind BMP-11 and/or activin, or other growth factors. The first amino acid sequence may be identical to or substantially as set out in SEQ ID NO:3 from amino acid (aa) 23 to aa 138 or from aa 19 to aa 134 in SEQ ID NO:1. The difference between SEQ ID NO:1 and SEQ ID NO:3 is that aa 64 of SEQ ID NO:1 is Ala, whereas the corresponding aa 68 in SEQ ID NO:3 is Arg. Additionally, other variances in the sequence of ActRIIB are possible, for example, aa 16 and aa 17 in SEQ ID NO:1 can be substituted with Cys and Ala, respectively. The first amino acid sequence may comprise at least 20, 30, 40, 50, 60, 70, 80, 90, 100, 110, or 120 contiguous amino acids from aa 23 and aa 138 of SEQ ID NO:3 or aa 19 and aa 134 of SEQ ID NO:1. Such a sequence can be truncated so long as the truncated sequence is capable of specifically binding GDF-8. Binding to GDF-8 can be assayed using methods known in the art or as described in Examples 5 and 6.

The second amino acid sequence is derived from the constant region of an antibody, particularly the Fc portion, or is a mutation of such a sequence. The second amino acid sequence may be derived from the Fc portion of an IgG. The Fc portion may be derived from IgG that is IgG₁, IgG₄, or another IgG isotype. In a particular embodiment, the second amino acid sequence comprises the Fc portion of human IgG₁ as set forth in SEQ ID NO:3 (amino acids 148 to 378), wherein the Fc portion of human IgG₁ has been modified to minimize the effector function of the Fc portion. Such modifications include changing specific amino acid residues which might alter an effector function such as Fc receptor binding (Lund et al. (1991) J. Immun. 147:2657-2662 and Morgan et al. (1995) Immunology 86:319-324), or changing the species from which the constant region is derived. Antibodies may have mutations in the C_{H}2 region of the heavy chain that reduce effector function, *i.e*., Fc receptor binding and complement activation. For example, antibodies may have mutations such as those described in U.S. Patent Nos. 5,624,821 and 5,648,260. In the IgG₁ or IgG₂ heavy chain, for example, such mutations may be made at amino acid residues corresponding to amino acids 234 and 237 in the full-length sequence of IgG₁ or IgG₂. Antibodies may also have mutations that stabilize the disulfide bond between the two heavy chains of an immunoglobulin, such as mutations in the hinge region of IgG₄, as disclosed in Angal et al. (1993) Mol. Immunol. 30:105-108:

The second amino acid sequence may be linked to the C-terminus or the N-terminus of the first amino acid sequence, with or without being linked by a linker sequence. The exact length and sequence of the linker and its orientation relative to the linked sequences may vary. The linker may be, for example, (Gly-Ser)₂ (SEQ ID NO:5). The linker may comprise 2, 10, 20, 30, or more amino acids and is selected based on properties desired such as solubility, length and steric separation, immogenicity, etc. The linker may comprise a sequence of a proteolytic cleavage site, such as the enterokinase cleavage site Asp-Asp-Asp-Lys (SEQ ID NO:6), or other functional sequences useful, for example, for purification, detection, or modification of the fusion protein.

It will be understood by one of ordinary skill in the art that certain amino acids in a sequence of any protein may be substituted for other amino acids without adversely affecting the activity of the protein. It is thus contemplated that various changes may be made in the amino acid sequences the sequence of the ActRIIB fusion polypeptides described herein, or DNA sequences encoding such polypeptides, without appreciable loss of their biological activity or utility. The biological activity of ActRIIB can be measured as described in Examples 6-10. Such changes may include, but are not limited to, deletions, insertions, truncations, and substitutions.

Additional fusions of any of ActRIIB fusion polypeptides described herein to amino acid sequences derived from other proteins may be constructed. Desirable fusion sequences may be derived from proteins having biological activity different from that of ActRIIB, for example, cytokines, growth and differentiation factors, enzymes, hormones, other receptor components, etc. Also, ActRIIB fusion polypeptides, may be chemically coupled, or conjugated, to other proteins and pharmaceutical agents. Such modification may be designed to alter the pharmacokinetics and/or biodistribution of the resultant composition.

The ActRIIB fusion polypeptides described herein can be glycosylated, pegylated, or linked to another nonproteinaceous polymer. For instance, the presently disclosed ActRIIB fusion polypeptides may be linked to one of a variety of nonproteinaceous polymers, *e.g*., polyethylene glycol, polypropylene glycol, or polyoxyalkylenes, in the manner set forth in U.S. Patent Nos. 4,640,835; 4,496,689; 4,301,144; 4,670,417; 4,791,192; or 4,179,337. The ActRIIB fusion polypeptides are chemically modified by covalent conjugation to a polymer to increase their circulating half-life, for example. Exemplary polymers, and methods to attach them to peptides, are also shown in U.S. Patent Nos. 4,766,106; 4,179,337; 4,495,285; and 4,609,546.

The ActRIIB fusion polypeptides described herein may be modified to have an altered glycosylation pattern (*i.e*., altered from the original or native glycosylation pattern). As used herein, "altered" means having one or more carbohydrate moieties deleted, and/or having one or more glycosylation sites added to the original sequence. Addition of glycosylation sites to the presently disclosed modified ActRIIB may be accomplished by altering the amino acid sequence to contain glycosylation site consensus sequences well known in the art. Another means of increasing the number of carbohydrate moieties is by chemical or enzymatic coupling of glycosides to the amino acid residues. These methods are described in WO 87/05330, and in Aplin et al. (1981) Crit. Rev. Biochem. 22:259-306. Removal of any carbohydrate moieties present on ActRIIB may be accomplished chemically or enzymatically as described by Hakimuddin et al. (1987) Arch. Biochem. Biophys. 259:52; Edge et al. (1981) Anal. Biochem. 118:131 and by Thotakura et al. (1987) Meth. Enzymol. 138:350.

The ActRIIB fusion polypeptides described herein may also be tagged with a detectable or functional label. Detectable labels include radiolabels such as ¹³¹I or ⁹⁹Tc, which may be attached to ActRIIB fusion polypeptides described herein using conventional chemistry known in the art. Labels also include enzyme labels such as horseradish peroxidase or alkaline phosphatase. Labels further include chemical moieties such as biotin, which may be detected via binding to a specific cognate detectable moiety, *e.g*., labeled avidin.

One of skill in the art will recognize that the ActRIIB fusion polypeptides described herein may be used to detect, measure, and inhibit proteins other than GDF-8, BMP-11, and activin. Nonlimiting examples of such proteins, for example, sequences of GDF-8 derived from various species (orthologues), are described in the present specification.

### III. Nucleic Acids, Cloning and Expression Systems

The present disclosure provides an isolated nucleic acid encoding a soluble ActRIIB that can be utilized in the methods of the present invention. The nucleic acid comprises a coding sequence for at least one ActRIIB fusion polypeptide as described herein. In one embodiment, the nucleic acid comprises the sequence, set forth in SEQ ID NO:4. The nucleic acid sequence may encode amino acids sequences from aa 23 and aa 138 of SEQ ID NO:3 or from aa 19 and aa 134 of SEQ ID NO:1.

The disclosure also provides constructs in the form of plasmids, vectors, transcription or expression cassettes which comprise at least one nucleic acid of the invention as above.

The disclosure also provides a host cell, which comprises one or more constructs as above. A nucleic acid encoding a fusion protein comprising the amino acid sequence of SEQ ID No.3 is itself an aspect of the present invention. Production of the encoded ActRIIB fusion polypeptides may be achieved by expression recombinant host cells containing the nucleic acid under appropriate culturing conditions. Following expression, an ActRIIB fusion polypeptide is isolated and/or purified using any suitable technique, then used as appropriate. Exemplary procedures for expression and purification are presented in Examples 3 and 4.

Specific ActRIIB fusion polypeptides and encoding nucleic acid molecules and vectors described herein may be obtained, isolated and/or purified, *e.g*., from their natural environment, in substantially pure or homogeneous form, or in the case of nucleic acid, free or substantially free of nucleic acid or genes origin other than the sequence encoding a polypeptide with the required function. Nucleic acids, according to the present invention, may comprise DNA or RNA and may be wholly or partially synthetic. Reference to a nucleotide sequence as set out herein encompasses a DNA molecule with the specified sequence, and encompasses a RNA molecule with the specified sequence in which U is substituted for T, unless context requires otherwise.

Described herein are sequences that are at least 100, 200, 300, 400, 500, 600, 700, 800, 900, or 1000 nucleotides long and hybridize under stringent hybridization conditions to the nucleic acid set forth in SEQ ID NO:4.

Systems for cloning and expression of a polypeptide in a variety of different host cells are well known. Suitable host cells include bacteria, mammalian cells, and yeast and baculovirus systems. Mammalian cell lines available in the art for expression of a heterologous polypeptide include Chinese hamster ovary cells, HeLa cells, baby hamster kidney cells, NS0 mouse melanoma cells and many others. A common bacterial host is *E. coli*. For other cells suitable for producing ActRIIB fusion polypeptides, see Gene Expression Systems, Academic Press (Fernandez et al. eds. 1999). Any cell line compatible with the present invention may be used to produce the ActRIIB fusion polypeptides described herein.

Suitable vectors can be chosen or constructed, containing appropriate regulatory sequences, including promoter sequences, terminator sequences, polyadenylation sequences, enhancer sequences, marker genes and other sequences as appropriate. Vectors may be plasmids or viral, *e.g*., phage, or phagemid, as appropriate. For further details see, for example, Sambrook et al. (1989) Molecular Cloning: A Laboratory Manual, 2nd ed., Cold Spring Harbor Laboratory Press. Many known techniques and protocols for manipulation of nucleic acid, for example, in preparation of nucleic acid constructs, mutagenesis, sequencing, introduction of DNA into cells and gene expression, and analysis of proteins, are described in detail in Current Protocols in Molecular Biology, 2nd ed., John Wiley & Sons (Ausubel et al eds. 1992).

Thus, a further aspect of the present invention is a host cell containing a nucleic acid encoding a fusion protein comprising the amino acid sequence of SEQ ID No. 3. Such nucleic acid may be introduced into a host cell. The introduction may employ any suitable technique. For eukaryotic cells, suitable techniques may include calcium phosphate transfection, DEAE-Dextran, electroporation, liposome-mediated transfection and transduction using retrovirus or other virus, *e.g*., vaccinia or, for insect cells, baculovirus. For bacterial cells, suitable techniques may include calcium chloride transformation, electroporation and transfection using bacteriophage.

The introduction may be followed by causing or allowing expression from the nucleic acid, *e.g.*, by culturing host cells under conditions appropriate for expression of the nucleic acid.

### IV. Methods for Identifying Inhibitors

Yet another aspect of the invention provides a method of identifying inhibitors of GDF 8. Appropriate screening assays, *e.g*., ELISA-based assays, are known in the art. In such a screening assay, a first binding mixture is formed by combining an ActRIIB fusion polypeptide and a ligand, *e.g.*, GDF-8, BMP-11, activin; and the amount of binding in the first binding mixture (M₀) is measured. A second binding mixture is also formed by combining an ActRIIB fusion polypeptide, the ligand, and the compound or agent to be screened, and the amount of binding in the second binding mixture (M₁) is measured. The amounts of binding in the first and second binding mixtures are then compared, for example, by calculating the M₁/M₀ ratio. The compound or agent is considered to be capable of inhibiting ActRIIB-mediated cell signaling if a decrease in binding in the second binding mixture as compared to the first binding mixture is observed. The formulation and optimization of binding mixtures is within the level of skill in the art, such binding mixtures may also contain buffers and salts necessary to enhance or to optimize binding, and additional control assays may be included in the screening assay of the invention.

Compounds found to reduce the ActRIIB fusion polypeptide-ligand binding by at least about 10% (*i.e.*, M₁/M₀<0.9), preferably greater than about 30%, may thus be identified and then, if desired, secondarily screened for the capacity to inhibit GDF-8 activity in other assays, such as the ActRIIB binding assay, and other cell-based and *in vivo* assays as described in Examples 5-12.

### V. Methods of Treating Disease and Other Uses

The presently disclosed ActRIIB fusion polypeptides are soluble and possess pharmacokinetic properties that make them suitable as therapeutic agents, *i.e.,* useful to prevent, diagnose, or treat various medical disorders in animals, and especially, humans. In certain embodiments, the circulatory half-life of the ActRIIB fusion polypeptide exceeds 5, 7, 10, or 14 days.

The ActRIIB fusion polypeptides can be used to inhibit one or more activities of GDF-8 associated with muscle and/or bone disorders. Inhibition of GDF-8 activity can be measured in pGL3(CAGA)₁₂ reporter gene assays (RGA) as described in Thies et al. (Growth Factors (2001) 18:251-259) or as illustrated in Example 6.

The medical disorder being diagnosed, treated, or prevented by the presently disclosed ActRIIB fusion polypeptides is a muscle or neuromuscular disorder; an adipose tissue disorder such as obesity; type 2 diabetes; impaired glucose tolerance; metabolic syndromes (*e.g*., syndrome X); insulin resistance induced by trauma such as burns or nitrogen imbalance; or bone degenerative disease such as osteoporosis.

The presently disclosed ActRIIB fusion polypeptides may also be used in therapies to repair damaged muscle, *e.g*., myocardium, diaphragm, etc. Exemplary disease and disorders further include muscle and neuromuscular disorders such as muscular dystrophy (including Duchenne's muscular dystrophy); amyotrophic lateral sclerosis (ALS), muscle atrophy; organ atrophy; frailty; carpal tunnel syndrome; congestive obstructive pulmonary disease; and sarcopenia, cachexia and other muscle wasting syndromes

Other medical disorders being diagnosed, treated, or prevented by the presently disclosed ActRIIB fusion polypeptides are disorders associated with a loss of bone, which include osteoporosis, especially in the elderly and/or postmenopausal women; glucocorticoid-induced osteoporosis; osteopenia; osteoarthritis; and osteoporosis-related fractures. Other target metabolic bone diseases and disorders include low bone mass due to chronic glucocorticoid therapy, premature gonadal failure, androgen suppression, vitamin D deficiency, secondary hyperparathyroidism, nutritional deficiencies, and anorexia nervosa. The ActRIIB fusion polypeptides are preferably used to prevent; diagnose, or treat such medical disorders in mammals, especially, in humans.

Compositions comprising the ActRIIB fusion polypeptides described herein are administered in therapeutically effective amounts. Generally, a therapeutically effective amount may vary with the subject's age, condition, and sex, as well as the severity of the medical condition in the subject. The dosage may be determined by a physician and adjusted, as necessary, to suit observed effects of the treatment. Generally, the compositions are administered so that polypeptides are given at a dose from 1 µg/kg to 20 mg/kg, 1 µg/kg to 10 mg/kg, 1 µg/kg to 1 mg/kg, 10 µg/kg to 1 mg/kg, 10 µg/kg to 100 µg/kg, 100 µg to 1 mg/kg, and 500 µg/kg to 1 mg/kg, or as described in Examples 10 and 11. The compositions may be given as a bolus dose, to maximize the circulating levels for the greatest length of time after the dose. Continuous infusion may also be used after the bolus dose.

The specification for the dosage unit of polypeptides described herein are dictated by and directly dependent on the unique characteristics of the active compound and the particular therapeutic effect to be achieved, and the limitations inherent in the art of compounding such an active compound for the treatment of individuals.

Toxicity and therapeutic efficacy can be determined by standard pharmaceutical procedures in cell cultures or experimental animals *e.g*., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀, Compositions that exhibit large therapeutic indices, are preferred.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized.

The therapeutically effective dose can be estimated initially from cell culture assays. A dose may be formulated in animal models to achieve a circulating plasma concentration range that includes the IC₅₀ (*i.e.*, the concentration of the therapeutic which achieves a half-maximal inhibition of symptoms) as determined in cell culture. Levels in plasma may be measured, for example, by high performance liquid chromatography. The effects of any particular dosage can be monitored by a suitable bioassay. Examples of suitable bioassays include DNA replication assays, transcription-based assays, GDF-8 binding assays, creatine kinase assays, assays based on the differentiation of pre-adipocytes, assays based on glucose uptake in adipocytes, and immunological assays.

The ActRIIB fusion polypeptides described herein may be used to detect the presence of proteins belonging to the TGF-β superfamily, such as BMP-11 and GDF-8, *in vivo* or *in vitro.* By correlating the presence or level of these proteins with a medical condition, one of skill in the art can diagnose the associated medical condition. The medical conditions that may be diagnosed by the presently disclosed ActRIIB fusion polypeptides are set forth above.

Such detection methods are well known in the art and include ELISA, radioimmunoassay, immunoblot, Western blot, immunofluorescence, immunoprecipitation, and other comparable techniques. The polypeptides may further be provided in a diagnostic kit that incorporates one or more of these techniques to detect a protein (*e.g*., GDF-8). Such a kit may contain other components, packaging, instructions, or other material to aid the detection of the protein and use of the kit.

Where the ActRIIB fusion polypeptides are intended for diagnostic purposes, it may be desirable to modify them, for example, with a ligand group (such as biotin) or a detectable marker group (such as a fluorescent group, a radioisotope or an enzyme). If desired, the ActRIIB fusion polypeptides may be labeled using conventional techniques. Suitable labels include fluorophores, chromophores, radioactive atoms, electron-dense reagents, enzymes, and ligands having specific binding partners. Enzymes are typically detected by their activity. For example, horseradish peroxidase can be detected by its ability to convert tetramethylbenzidine (TMB) to a blue pigment, quantifiable with a spectrophotometer. Other suitable binding partners include biotin and avidin or streptavidin, IgG and protein A, and the numerous receptor-ligand couples known in the art.

### VI. Pharmaceutical Compositions and Methods of Administration

Described herein are compositions suitable for administration to patients. The compositions typically comprise one or more ActRIIB fusion polypeptides described herein and a pharmaceutically acceptable excipient. As used herein, the phrase "pharmaceutically acceptable excipient" refers to any and all solvents, dispersion media, coatings, antibacterial and antifungal agents, isotonic and absorption delaying agents, and the like, that are compatible with pharmaceutical administration. The use of such media and agents for pharmaceutically active substances is well known in the art. The compositions may also contain other active compounds providing supplemental, additional, or enhanced therapeutic functions. The pharmaceutical compositions may also be included in a container, pack, or dispenser together with instructions for administration.

A pharmaceutical composition of the invention is formulated to be compatible with its intended route of administration. Methods to accomplish the administration are known to those of ordinary skill in the art. The administration may, for example, be intravenous, intraperitoneal, intramuscular, intracavity, subcutaneous or transdermal. It may also be possible to obtain compositions that may be topically or orally administered.

Solutions or suspensions used for intradermal or subcutaneous application typically include one or more of the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates; and agents for the adjustment of tonicity such as sodium chloride or dextrose. The pH can be adjusted with acids or bases, such as hydrochloric acid or sodium hydroxide. Such preparations may be enclosed in ampoules, disposable syringes or multiple dose vials made of glass or plastic.

Pharmaceutical compositions suitable for injection include sterile aqueous solutions or dispersions and sterile powders for the extemporaneous preparation of sterile injectable solutions or dispersion. For intravenous administration, suitable carriers include physiological saline, bacteriostatic water, Cremophor™ EL (BASF, Parsippany, NJ) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and gelatin.

Oral compositions generally include an inert diluent or an edible carrier. They can be enclosed in gelatin capsules or compressed into tablets. For the purpose of oral therapeutic administration, the ActRIIB fusion polypeptides can be incorporated with excipients and used in the form of tablets, troches, or capsules. Pharmaceutically compatible binding agents, and/or adjuvant materials can be included as part of the composition. The tablets, pills, capsules, troches, and the like can contain any of the following ingredients, or compounds of a similar nature; a binder such as microcrystalline cellulose, gum tragacanth or gelatin; an excipient such as starch or lactose, a disintegrating agent such as alginic acid, Primogel™, or corn starch; a lubricant such as magnesium stearate or Sterotes™; a glidant such as colloidal silicon dioxide; a sweetening agent such as sucrose or saccharin; or a flavoring agent such as peppermint, methyl salicylate, or orange flavoring.

For administration by inhalation, the ActRIIB fusion polypeptides are delivered in the form of an aerosol spray from pressured container or dispenser which contains a suitable propellant, *e.g*., a gas such as carbon dioxide, or a nebulizer.

Systemic administration can also be by transmucosal or transdermal means. For example, in the case of ActRIIB-Fc, compositions may be capable of transmission across mucous membranes (*e.g*., intestine, mouth, or lungs) via the FcRn receptor-mediated pathway (U.S. Patent No. 6,030,613). Transmucosal administration can be accomplished, for example, through the use of lozenges, nasal sprays, inhalers, or suppositories. For transdermal administration, the active compounds are formulated into ointments, salves, gels, or creams as generally known in the art. For transmucosal or transdermal administration, penetrants appropriate to the barrier to be permeated are used in the formulation. Such penetrants are generally known in the art, and include, for example, detergents, bile salts, and fusidic acid derivatives.

The presently disclosed ActRIIB fusion polypeptides can prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. Liposomal suspensions containing the presently disclosed ActRIIB fusion polypeptides can also be used as pharmaceutically acceptable carriers. These can be prepared according to methods known to those skilled in the art, for example, as described in U.S. Patent No. 4,522,811.

It is may be advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

Nucleic acids encoding ActRIIB fusion polypeptides, such as the nucleic acids described above, can be introduced to a cell within tissue, an organ, or an organism so that the encoded polypeptides can then be expressed. This methodology may be useful, for example, in evaluating effects of ActRIIB fusion polypeptides on individual tissues and organs. The nucleic acid encoding an ActRIIB fusion polypeptide may be linked to a tissue-specific expression control sequence, *e.g*., muscle-specific promoter sequence such as the myosin promoter or the desmin promoter, the muscle-specific enhancer elements such as the muscle creatine kinase enhancer. Those of skill in the art will recognize that specific polynucleotide sequences can be inserted into the viral or plasmid vectors that can be injected into a mammal systemically, or locally. Host cells may also be harvested, and a nucleic acid encoding an ActRIIB fusion polypeptide may be transfected into such cells *ex vivo* for subsequent reimplantation using methods known in the art. Nucleic acids may be also transfected into a single cell embryo to create a transgenic animal as described in Gene Expression Systems, Academic Press (Fernandez et al. eds. 1999).

The following examples illustrate some embodiments and aspects of the invention.

### EXAMPLES

### Example 1: Purification of GDF-8

Conditioned media from a selected cell line expressing recombinant human GDF-8 protein (mature GDF-8 and GDF-8 propeptide) was acidified to pH 6.5 and applied to a 80 x 50 mm POROS™ HQ anion exchange column in tandem to a 80 x 50 mm POROS™ SP cation exchange column (PerSeptive Biosystems, Foster City, CA). The flow through was adjusted to pH 5.0 and applied to a 75 x 20 mm POROS™ SP cation exchange column (PerSeptive Biosystems) and eluted with a TFA/acetonitrile gradient. Fractions containing the GDF-8 latent complex, as confirmed by SDS-PAGE, were pooled, acidified with trifluoroacetic acid (TFA) to pH 2-3, then brought up to 200 ml with 0.1 % TFA to lower the viscosity. The pool was then applied to a 250 x 21.2 mm C₅ column (Phenomenex, Torrance, CA) preceded by a 60 x 21.2 mm guard column (Phenomenex) and eluted with a TFA/acetonitrile gradient, to separate mature GDF-8 from GDF-8 propeptide. Pooled fractions containing mature GDF-8 were concentrated by lyophilization to remove the acetonitrile and 20 ml of 0.1% TFA was added. The sample was then applied to a 250 x 10 mm C₅ column (Phenomenex) heated to 60°C to aid in separation. This was repeated until further separation could no longer be achieved. Fractions containing mature GDF-8 were then pooled and brought up to 40% acetonitrile and applied to a 600 x 21.2 BioSep™ S-3000 size exclusion column (Phenomenex) preceded by a 60 x 21.2 guard column. Fractions containing purified mature GDF-8 and the GDF-8 propeptide were separately pooled and concentrated for use in subsequent experiments.

On SDS-PAGE, purified mature GDF-8 migrated as a broad band at 25 kDa under nonreducing conditions and 13 kDa under reducing conditions. Under reducing and non-reducing conditions, BMP-11 propeptide migrated at around 35 kDa. A similar SDS-PAGE profile has been reported for murine GDF-8 by McPherron et al. (Proc. Natl. Acad. Sci. U.S.A. (1997) 94:12457-12461) and reflects the dimeric nature of the mature protein. The GDF-8 propeptide migrated at about 35 kDa on reducing SDS-PAGE. Active mature BMP-11 dimer was purified from conditioned media from a cell line expressing recombinant human BMP-11 in the same manner. The conditioned media was loaded onto a 10 ml TALON™ column (Clonetech, Palo Alto, CA). The bound protein was eluted with 50 mM Tris pH 8.0/1 M NaCI/500 mM imidazole. Fractions containing the BMP-11 complex were pooled and acidified with 10% trifluoroacetic acid to a pH of 3. The BMP-11 complex pool was applied to a 250 x 4.6 mm Jupiter C4 column (Phenomenex), which was heated to 60°C for better separation of the mature BMP-11 and BMP-11 propeptide. BMP-11 was eluted with a TFA/acetonitrile gradient. The fractions containing BMP-11 were concentrated by lyophilization to remove the acetonitrile.

### Example 2: Characteristics of Purified Recombinant Human GDF-8

50 µg of each purified mature GDF-8 and purified GDF-8 propeptide were mixed and dialyzed into 50 mM sodium phosphate, pH 7.0, and chromatographed on a 300 x 7.8 mm BioSep™ S-3000 size exclusion column (Phenomenex). Molecular weight of the mature GDF-8/propeptide complex was determined from elution time, using molecular weight standards (Bio-Rad Laboratories, Hercules, CA) chromatographed on the same column.

When purified GDF-8 propeptide was incubated with purified mature GDF-8 at neutral pH, the two proteins appeared to complex, as indicated by the size exclusion profile. The primary protein peak eluted at 12.7 minutes had an estimated molecular weight of 78 kDa from molecular weight standards (Bio-Rad Laboratories) chromatographed on the same column. The size of the complex was most consistent with one dimer of the mature GDF-8 associating with two monomers of propeptide.

To confirm this observation, a preparation containing both mature GDF-8 and GDF-8 propeptide was incubated with or without 100 mM 1-Ethyl 3-(3-dimethylaminopropyl)carbodiamide hydrochloride (EDC, Pierce Chemical, Rockford, IL) for 1 hour at room temperature, acidified with HCl to pH 2-3, and concentrated with a Micron-10 Amicon concentrator (Millipore, Bedford, MA) for SDS-PAGE, using a tricine buffered 10% acrylamide gel. Proteins were visualized by Coomassie™ blue staining of SDS-PAGE.

### Example 3: Expression of ActRIIB-Fc in CHO Cells

A full-length human ActRIIB cDNA was used to PCR-clone the extracellular domain (excluding the sequence encoding the signal peptide). The primers used were flanked by Spel (5') and Notl (3') sites. Following PCR amplification, this PCR fragment was cloned into the SpeI/NotI sites of the expression plasmid pHTop-HBML/EKFc. The open reading frame encodes: honeybee mellitin leader (amino acids 1 to-21 of SEQ ID NO:3); human ActRIIB extracellular domain (amino acids 23 to 138 of SEQ ID NO:3); enterokinase cleavage site (DDDK, SEQ ID NO:6); and human IgG₁ Fc fragment (amino acids 148 to 378 of SEQ ID NO:3). As a result of the insertion of the Spel site, there was an addition of Thr-22 in the sequence.

A CHO stable cell line stably transfected to express the above ActRIIB-Fc was obtained by lipofectin transfection of the pHTop-HBML vector containing the ActRIIB-Fc construct into CHO/A2 cells. Transfected cells were selected in 0.1 µM methotrexate. Western blot analysis of conditioned media was used to identify the highest expressing clones.

The pHTop vector was derived from pED (Kaufman et al. (1991) Nucleic Acids Res. 19:4485-4490) by removing the majority of the adeno major late promoter and inserting six repeats of the *tet* operator as described in Gossen et al. (1992) Proc. Natl. Acad. Sci. U.S.A. 89:5547-5551.

The CHO/A2 cell line was derived from CHO DUKX B11 cells (Urlaub et al. (1980) Proc. Natl. Acad. Sci. U.S.A. 77:4216-4220) by stably integrating a transcriptional activator, a fusion protein between the tet repressor fused to the herpes virus VP16 transcriptional domain (Gossen et al. (1992) Proc. Natl. Acad. Sci. U.S.A. 89:5547-5551).

### Example 4: Purification of ActRIIB-Fc

Raw material concentrated from conditioned medium was purified by rProtein A Sephadex Fast Flow™ (XK26/4.5 cm, 23.8 ml; Pharmacia, Piscataway, NJ) to 99% purity as determined by size exclusion chromatography as follows. Frozen conditioned medium was thawed at 37°C water bath and filtered through 0.22 µm filters. Four parts of the filtered solution was mixed with one part of Protein A loading buffer (0.65 M Na₂SO₄ 20 mM sodium citrate, 20 mM boric acid, 20 mM Na₂HPO₄, pH 9.0) and ran over the protein A column at room temperature. ActRIIB-Fc was eluted off the column using Protein A eluting buffer (0.15 M NaCI, 20 mM citric acid, pH 2.5) with gradient or step up at pH around 4-5, and the peak was collected and neutralized to pH 7.0 by adding 25% neutralization buffer (0.05 M Na₂HPO₄, 0.15 M NaCl, pH 7.2). The fractions were evaluated by size exclusion chromatography and SDS-PAGE, and then pooled and stored at 4°C. The purified protein was formulated into PBS by Sephadex™ G-25 desalting column (XK50/13.4 cm, 236 ml, Pharmacia), and then filtered through a 0.22 µm filer and stored at 4°C.

### Example 5: Binding Properties of Purified GDF-8 and BMP-11 in ActRIIB-Fc Binding Assay

The GDF-8 latent complex was biotinylated at a ratio of 20 moles of EZ-link™ Sulfo-NHS-Biotin (Pierce Chemical, Cat. No. 21217) to 1 mole of the GDF-8 complex for 2 hours on ice, inactivated with 0.5% TFA, and subjected to chromatography on a C4 Jupiter 250 x 4.6 mm column (Phenomenex) to separate mature GDF-8 from GDF-8 propeptide. Biotinylated mature GDF-8 fractions eluted with a TFA/acetonitrile gradient were pooled, concentrated and quantified by MicroBCA™ protein Assay Reagent Kit (Pierce Chemical, Cat. No. 23235).

Biotinylated mature BMP-11 was prepared from BMP-11 latent complex in the same manner as described above. Recombinant ActRIIB-Fc, prepared as described in Examples 3 and 4, was coated on 96-well flat-bottom assay plates (Costar, NY, Cat. No. 3590) at 1 µg/ml in 0.2 M sodium carbonate buffer (pH 10) overnight at 4°C. Plates were then blocked with 1 mg/ml bovine serum albumin and washed following standard ELISA protocol. 100 µl aliquots of biotinylated GDF-8 or BMP-11 at various concentrations were added to the blocked ELISA plate, incubated for 1 hr and washed. The amount of bound GDF-8 or BMP-11 was detected by Streptavidin-Horseradish peroxidase (SA-HRP, BD PharMingen, San Diego, CA, Cat. No.13047E) followed by the addition of TMB (KPL, Gaithersburg, MD, Cat. No. 50-76-04). Colorimetric measurements were done at 450 nM in a Molecular Devices microplate reader.

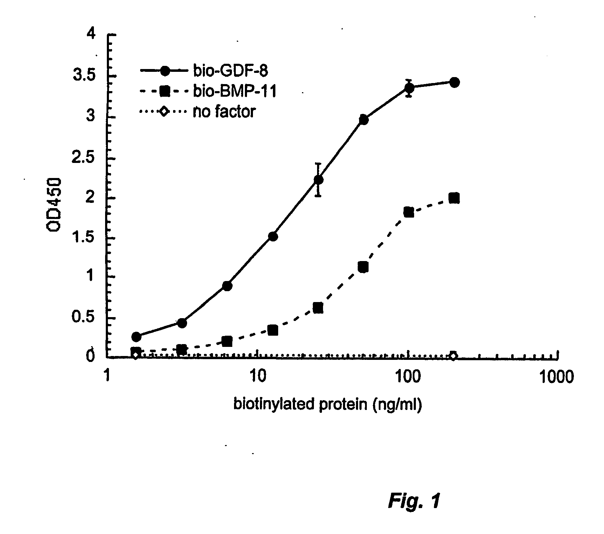

As shown in Figure 1, biotinylated GDF-8 and BMP-11 bound to ActRIIB-Fc, with an ED₅₀ of 15 ng/ml and 40 ng/ml, respectively.

### Example 6: Inhibitory Activity of ActRIIB-Fc in Reporter Gene Assay

To demonstrate the activity of ActRIIB-Fc, a reporter gene assay (RGA) was developed using a reporter vector PGL3(CAGA)₁₂ sequence coupled luciferase. The CAGA sequence was previously reported to be a TGF-β responsive sequence within the promoter of the TGF-β induced gene PAI-1 (Denner et al. (1998) EMBO J. 17:3091-3100).

A reporter vector containing 12 CAGA boxes was made using the basic reporter plasmid PGL3 (Promega, Madison, WI). The TATA box and transcription initiation site from the adenovirus major later promoter (-35/+10) was inserted between the Bglll and Hindlll sites. Oligonucleotides containing 12 repeats of the CAGA boxes, AGCCAGACA, were annealed and cloned into the Xhol site. The human rhabdomyosarcoma cell line A204 (ATCC HTB-82) was transiently transfected with pGL3(CAGA)₁₂ using FuGENE™ 6 transfection reagent (Boehringer Manheim, Germany). Following transfection, cells were cultured on 48 well plates in McCoy's 5A medium supplemented with 2 mM glutamine, 100 U/ml streptomycin, 1000 µg/ml penicillin and 10% fetal calf serum for 16 hrs. Cells were then treated with or without 10 ng/ml GDF-8 and various concentration of ActRIIB-Fc in McCoy's 5A media with glutamine, streptomycin, penicillin, and 1 mg/ml bovine serum albumin for 6 hrs at 37°C. Luciferase was quantified in the treated cells using the Luciferase Assay System (Promega).

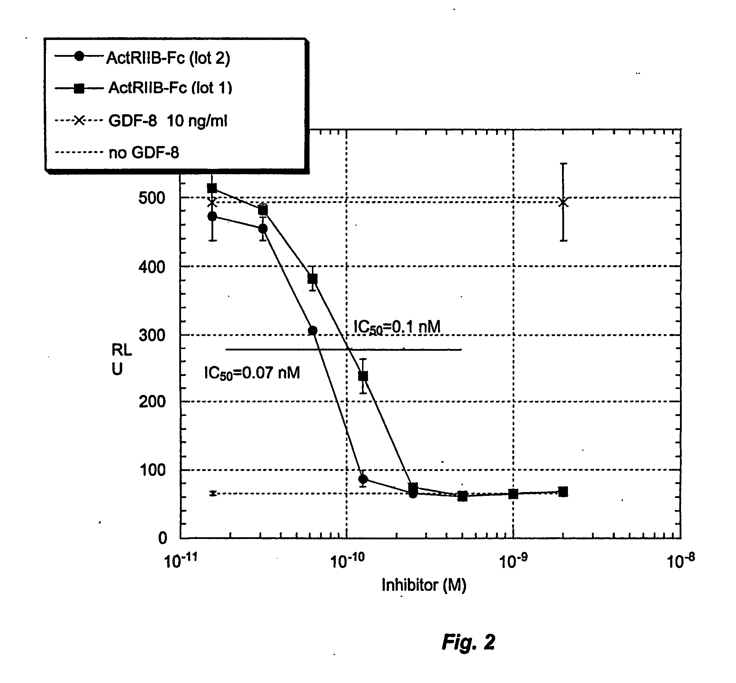

Two independently purified lots of ActRIIB showed an IC₅₀ from 0.07 to 0.1 nM in the above reporter gene assay (Figure 2).

### Example 7: Pharmacokinetics

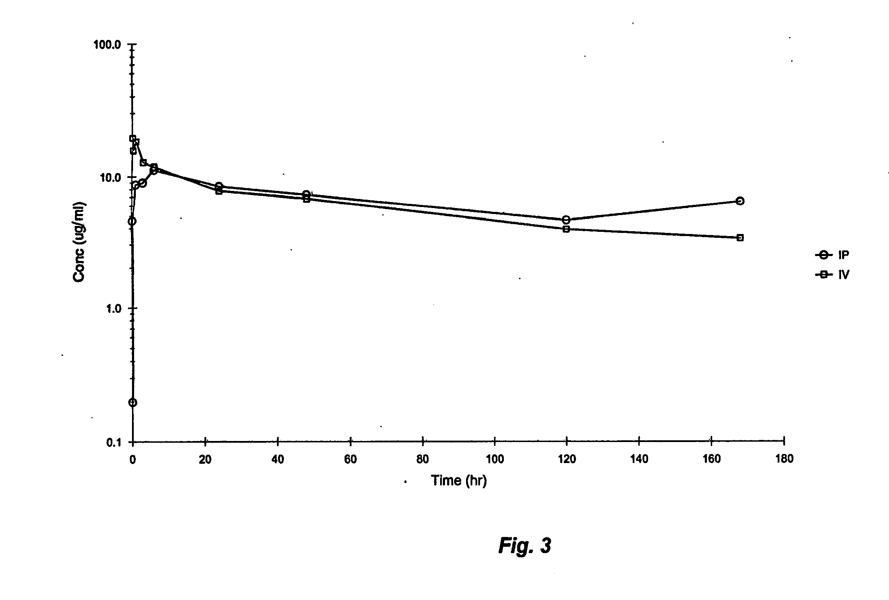

The pharmacokinetics (PK) of ActRIIB-Fc was evaluated in C57B6/SCID mice (The Jackson Laboratory, Bar Harbor, ME) at a dose of 1 mg/kg as a single intravenous (IV) or intraperitoneal (IP) administration. ActRIIB-Fc, produced and purified as described in Examples 3 and 4, was radiolabeled using the iodogen method (Protein Pharmacokinetics and Metabolism, Plenum Press, New York, NY (Ferraiolo et al. eds. 1992)). The animals received a mixture of unlabeled and ¹²⁵I labeled ActRIIB-Fc at the dose listed above and serum concentrations were determined based on ¹²⁵I radioactivity in the serum and the specific activity of the injected dose. Figure 3 shows the serum concentration based on TCA-precipitated counts versus time for ActRIIB-Fc administered either IV or IP. Absorption from IP injection was complete, and bioavailability was close to 100% within the first 180 hr post injection; the initial volume distribution matched mouse plasma volume (50 ml/kg); peak serum concentration was 11 µg/ml (IP, at 6 hr post injection) and 19.4 µg/ml (IV); half-life during the terminal elimination phase was about 5 days.

### Example 8: In vivo Effect of ActRIIB-Fc

In order to determine if ActRIIB increases muscle mass in adult mice, an *in vivo* study on was conducted with seven-week-old female C57B6/SCID (The Jackson Laboratory). Mice were weighed and evenly distributed with respect to body weight into groups of eight. During a four-week study, each group received a weekly intraperitoneal injection of the following: ActRIIB-Fc (60 mg/kg, 3 mg/kg, or 60 µg/kg), mouse monoclonal anti-GDF-8 antibody JA16 (60 mg/kg), or PBS buffer (vehicle control). JA16 was chosen because this antibody is specific for GDF-8, and was shown to inhibit the muscle-downregulatory activity of GDF-8 *in vivo,* in a separate study (U.S. Patent App. Pub. No. 20030138422). Animals were assessed for gain in lean body mass by subjecting them to dexascan analysis before and after the treatment period. Muscle mass was assessed by dissecting and weighing the gastrocnemius and quadriceps. The peri-uterine fat pad was also removed and weighed. Spleen and thymus weights were also measured.

The results of this study indicated that ActRIIB-Fc significantly inhibited GDF-8 activity *in vivo* resulting in increased muscle mass. As anticipated, mice administered JA16 exhibited slightly higher body and skeletal muscle weights and had a statistically significant (p=0.05) increase in quadriceps weights (Table 4). The treatments with 60 and 3 mg/kg ActRIIB-Fc were surprisingly significantly more effective as compared to the JA16 antibody. The groups administered 60 mg/kg ActRIIB-Fc and 3 mg/kg ActRIIB-Fc had about 3 and 2 times increased body weights respectively as compared to the controls (Table 1). These increases were first observed after one dose. The quadriceps muscle weights, as absolute weights, were increased in the mice administered 60 and 3 mg/kg ActRIIB-Fc - (Table 3). The gastrocnemius muscles, as absolute weights, were increased in mice administered 60 mg/kg JA16 and 60 or 3 mg/kg ActRIIB-Fc (Table 3). As a percent of body weight, quadriceps muscle weights were increased in the same three treatment groups compared to controls (Table 4). Also, as a percent of body weight, the gastrocnemius weight was increased in the mice treated with 60 mg/kg ActRIIB-Fc (Table 4).

**TABLE 1: Terminal Body Weights**

| | **Control** | **JA16 60 mg/kg** | **ActRIIB 60 mg/kg** | **ActRIIB 3 mg/kg** | **ActRIIB 60 µg/kg** |
|---|---|---|---|---|---|
| **Body Weight (g)± SD** | 20.2±1.76 | 20.9±1.12 | 25.0 ± 1.90* | 22.5 ± 2.35* | 20.8 ± 1.97 |

| | | | | | |
|---|---|---|---|---|---|
| *= Group Difference at p=0.05 compared to controls | | | | | |

**TABLE 2: Absolute Weight Gain**

| | **Control** | **JA16 60 mg/kg** | **ActRIIB 60 mg/kg** | **ActRIIB 3 mg/kg** | **ActRIIB 60 µg/kg** |
|---|---|---|---|---|---|
| **Body Weight (g)±SD** | 1.99 ±.1.123 | 2.62 ± 1.007 | 6.23 ± 1.126* | 4.28 ± 1.748* | 1.24 ±1.010 |

| | | | | | |
|---|---|---|---|---|---|
| *= Group Difference at p=0.05 compared to controls | | | | | |

**TABLE 3: Absolute Organ Weights (g)**

| | **Control** | **JA16 60 mg/kg** | **ActRIIB 60 mg/kg** | **ActRIIB 3 mg/kg** | **ActRIIB 60 µg/kg** |
|---|---|---|---|---|---|
| **Spleen** | 0.044 | 0.025* | 0.060 | 0.071 | 0.059 |
| **Thymus** | 0.0342 | 0.178* | 0.0260 | 0.0333 | 0.0344 |
| **Quadriceps** | 0.151 | 0.171 | 0.232** | 0.193** | 0.159 |
| **Gastroc.** | 0.111 | 0.123* | 0.156** | 0.133* | 0.112 |

| | | | | | |
|---|---|---|---|---|---|
| * =Group Difference at p=0.05 compared to controls **=Group Difference at p=0.01 compared to controls | | | | | |

**TABLE 4: Organ Weights as Percent of Body Weight**

| | **Control** | **JA16 60 mg/kg** | **ActRIIB 60 mg/kg** | **ActRIIB 3 mg/kg** | **ActRIIB 60 µg/kg** |
|---|---|---|---|---|---|
| **Spleen** | 0.214 | 0.119* | 0.227 | 0.298 | 0.273 |
| **Thymus** | 0.1628 | 0.0850* | 0.0992 | 0.1391 | 0.1569 |
| **Quadriceps** | 0.749 | 0.820* | 0.926** | 0.861** | 0.768 |
| **Gastrocnemius** | 0.548 | 0.590 | 0.621** | 0.593 | 0.540 |

| | | | | | |
|---|---|---|---|---|---|
| * =Group Difference at p=0.05 compared to controls **=Group Difference at p=0.01 compared to controls | | | | | |

### Example 9: Dose-Dependent Effect of ActRIIB-Fc on Muscle Mass

To further investigate the effect of ActRIIB-Fc on muscle mass in adult mice, a study on was conducted with seven-week-old female C57B6/SCID (The Jackson Laboratory).. Mice were weighed and evenly distributed with respect to body weight into four groups of six (6 SCID, 6 C57 mice, and two control groups of 6 mice each). Each group received a weekly intraperitoneal injection of 60 mg/kg ActRIIB-Fc or PBS buffer (vehicle control) for one to four weeks. On day 29 of the study, animals were assessed for muscle mass was assessed by dissecting and weighing the gastrocnemius and quadriceps. The results of this study indicated that ActRIIB-Fc significantly inhibited GDF-8 activity *in vivo* resulting in increased muscle mass even after a single administration of ActRIIB as compared to the vehicle control. The quadriceps muscle weights, as absolute weights, were increased in both C57 and SCID mice by 21% to 60% (Table 5). Likewise, the gastrocnemius muscle mass, as absolute weights, was increased by 31 to 51% (Table 5).

**TABLE 5: Increase in Muscle Mass Following One or More Doses of ActRIIB-Fc**

| | **ActRIIB 1 dose** | **ActRIIB 2 doses** | **ActRIIB 4 doses** |
|---|---|---|---|
| **Quadriceps (SCID)** | 21% | 60% | 44% |
| **Gastrocnemius (SCID)** | 47% | 36% | 31% |
| **Quadriceps (C57)** | 41% | | 65% |
| **Gastrocnemius (C57)** | 37% | | 51% |

### Example 10: In vivo Role of GDF-8 in Trabecular Bone

Inhibition of GDF-8 increases muscle mass. Increased mechanical loading, either due to increased muscle activity or increased body weight, is associated with increased bone mass and bone density. Therefore, GDF-8 knockout (KO) mice were assessed for altered bone mass and microarchitecture: An initial assessment of adult mice showed that bone density in the spine of the KO mice was nearly two-fold higher than that of their wild-type littermates. This increase far exceeded what might have been expected to be solely due to the increased muscle mass in the GDF-8 KO mice.

High resolution microtomographic,imaging (µCT40, Scanco Medical, Switzerland) was used to assess the trabecular bone volume fraction and microarchitecture in the 5th lumbar vertebrae and distal femora and cortical bone geometry at the femoral mid-diaphysis of adult GDF-8 wildtype (WT) and KO mice. Specimens were taken from 9-10 month old GDF-8 KO and littermate controls (four mice of each genotype and sex). The entire vertebral body and femur were scanned using microcomputed tomography at 12 µm resolution. Regions of interest encompassing the trabecular bone of the vertebral body or the trabecular bone of the distal femoral metaphysis (i.e., secondary spongiosa) were identified using a semi-automated, contouring algorithm. The following parameters were computed using direct 3D assessments: bone volume fraction (%), trabecular thickness (µm), separation (µm) and number (1/mm). In addition, the connectivity density, an indicator of how well the trabecular network is connected, was assessed as well as cortical bone parameters at the middiaphyseal region in the femur, including total area, bone area, and cortical thickness.

Both male and female KO mice had dramatically increased trabecular bone density in the vertebral body compared to WT littermates (n=4, +93% and +70%, respectively, p<0.0001). This increased trabecular bone density was accompanied by a 14% increase in trabecular thickness (p=0.03), a 38% increase in trabecular number (p=0.0002), and a 10% decrease in trabecular separation (p=0.009). The combined effect of these changes in architecture and density resulted in a 3.4- and 1.7-fold increase in connectivity in male and female KO, respectively, compared to their WT littermates (p<0.0001). In addition, a rough measure of the level of mineralization of the trabecular bone indicated that the average mineral content of the trabeculae was 8% higher in the KO mice relative to the controls (p<0.0001). There is a hint that the effect is larger in male than female mice, but the sample size is too small to make definitive conclusions. Vertebral trabecular bone characteristics assessed by high-resolution microcomputed tomography are shown in Table 6.

In contrast to observations in the spine, male and female KO mice had lower trabecular bone density in the distal femur than WT littermates (n=4, p=0:05 for overall genotype effect) (Table 7). This decrement in bone density was more pronounced in female KO.than in male KO mice. GDF-8 KO mice had similar trabecular thickness as their WT littermates, but had fewer trabeculae and increased trabecular separation compared to littermate controls. However, although cortical thickness at the femoral midshaft was similar in male GDF-8 KO and their littermate controls, it was approximately 10% greater in the GDF-8 KO female mice than their WT littermates (n=4, p=0.04) (see Table 8). There were no differences in cortical bone area or bone area fraction between the two genotypes.

**TABLE 6: Vertebral Trabecular Bone Characteristics (Mean ± SEM)**

| | **Male WT** | **Male KO** | **Female WT** | **Female KO** |
|---|---|---|---|---|
| **Bone volume fraction (%)** | 23.3 ± 4.7 | 45.0 ± 5.5 | 27.5 ± 5.5 | 46.9 ± 10.8 |
| **Trabecular thickness (µm)** | 52 ± 3 | 58 ± 6 | 52 ± 5 | 61 ± 8 |
| **Trabecular separation (µm)** | 210 ± 21 | 145 ± 8 | 183 ± 21 | 169 ± 41 |
| **Trabecular number (1/mm)** | 4.6 ± 0.4 | 7.0 ± 0.4 | 5.2 ± 0.4 | 6.6 ± 1.3 |
| **Connectivity density (1/mm³)** | 137 ±15 | 470 ± 114 | 198 ± 29 | 339 ± 81 |
| **Degree of anisotropy** | 1.68 ± 0.08 | 1.29 ± 0.02 | 1.54 ± 0.12 | 1.34 ± 0.03 |

**TABLE 7: Characteristics of the Trabecular Bone in Distal Femoral Metaphysis (Mean ± SEM)**

| | **Male WT** | **Male KO** | **Female WT** | **Female KO** |
|---|---|---|---|---|
| **Bone volume fraction (%)** | 5.1 ± 1.8 | 2.9 ± 1.7 | 11.9 ± 7.0 | 54 ± 3.1 |
| **Trabecular thickness (µm)** | 68 ±1.2 | 68 ± 2.7 | 73 ± 7 | 63 ± 9 |
| **Trabecular separation (µm)** | 353 ± 16 | 472 ± 90 | 296 ± 73 | 464 ± 98 |
| **Trabecular number (1/mm)** | 2.84 ± 0.12 | 2.24 ± 0.51 | 3.46 ± 0 69 | 2.26 ± 0.57 |
| **Connectivity density (1/mm³)** | 5.9 ± 3.7 | 4.0 ± 6.9 | 31.5 ± 25.2 | 15.4 ± 15.1 |

**TABLE 8: Characteristics of the Cortical Bone at the Femoral Mid-Diaphysis (Mean ± SEM)**

| | **Male WT** | **Male KO** | **Female WT** | **Female KO** |
|---|---|---|---|---|
| **Bone Area (mm²)** | 5.1 ± 1.8 | 2.9 ± 1.7 | 11.9 ± 7.0 | 5.4 ± 3.1 |
| **Cortical Thickness (µm)** | 68 ± 1.2 | 68 ± 2.7 | 73 ± 7 | 63 ± 9 |
| **Bone Areal/Total Area (%)** | 353 ± 16 | 472 ± 90 | 296 ± 73 | 464 ± 98 |

### Example 11: Treatment of Muscle and Bone Degenerative Disorders

Inhibitors of GDF-8 such as, for example, ActRIIB fusion polypeptides are useful for treatments directed at increased muscle mass, and also for prevention and treatment of osteoporosis. In addition, Inhibition of GDF-8 may be useful in other instances where a bone anabolic effect is desired, such as augmentation of bone healing (i.e., fracture repair, spine fusion, etc.). The ActRIIB fusion polypeptides described herein are used to treat a subject at disease onset or having an established muscle or bone degenerative disease.

Efficacy of ActRIIB-Fc for treatment of bone disorders, *e.g*., osteoporosis, is confirmed using well-established models of osteoporosis. For example, ovariectomized mice have been used to test the efficacy of new osteoporosis drug treatments (Alexander et al. (2001) J. Bone Miri. Res. 16:1665-1673; and Anderson et al. (2001) J. Endocrinol. 170:529-537). Similar to humans, these rodents exhibit a rapid loss of bone hollowing ovariectomy, especially in cancellous bone. Outcome assessments are based on bone mineral density, biochemical markers of bone turnover in serum and urine, bone strength, and histology/histomorphometry.

In one study, normal and/or immune compromised female mice are ovariectomized at 12-16 weeks of age and allowed to lose bone for four to six weeks. Following this bone loss period, treatment with ActRIIB-Fc (IP injection) or vehicle is conducted for one to six months. The treatment protocol could vary, with testing of different doses and treatment regimens (*e.g*., daily, weekly, or bi-weekly injections). It is anticipated that untreated ovariectomized mice (or rats) would lose approximately 10-30% of bone density relative to intact (*i.*e., non-ovariectomized), age-matched mice. It is anticipated that mice treated with ActRIIB-Fc would have 10 to 50% greater bone mass and bone density than those mice receiving vehicle treatment, and moreover that this increase in bone density would be associated with increased bone strength, particularly in regions with a greater proportion of cancellous bone compared to cortical bone.

The goal of another study is to demonstrate that ActRIIB-Fc is effective in preventing the decline in bone mass, microarchitecture and strength associated with estrogen deficiency. Thus, the study has a similar design to the one described above, except that treatment with ActRIIB-Fc antibody would be initiated immediately after ovariectomy, rather than after the bone loss period. It is anticipated that mice treated with ActRIIB-Fc would lose significantly less bone mass following ovariectomy than mice treated with vehicle.

The ActRIIB fusion polypeptides are also used to prevent and/or to reduce severity and/or the symptoms of the disease. It is anticipated that the ActRIIB fusion polypeptides would be administered as a subcutaneous injection as frequently as once per day and as infrequently as once per month. Treatment duration could range from one month and several years.

To test the clinical efficacy of ActRIIB-Fc in humans, postmenopausal women with low bone mass are identified by bone density testing and randomized to a treatment group. Treatment groups include a placebo group and one to three groups receiving antibody (different doses). Individuals are followed prospectively for one to three years to assess changes in biochemical markers of bone turnover, changes in bone mineral density, and the occurrence of fragility fractures. It is anticipated that individuals receiving treatment would exhibit an increase in bone mineral density in the proximal femur and lumbar spine of 2-30% relative to baseline, and would have a decreased incidence of fragility fractures. It is anticipated that biochemical markers of bone formation would increase.

The polypeptides are administered as the sole active compound or in combination with another compound or composition. When administered as the sole active compound or in combination with another compound or composition, the dosage is preferably from approximately 1 µg/kg and 20 mg/kg, depending on the severity of the symptoms and the progression of the disease. The appropriate effective dose is selected by a treating clinician from the following ranges: 1 µg/kg to 20 mg/kg, 1 µg/kg to 10 mg/kg, 1 µg/kg to 1 mg/kg, 10 µg/kg to 1 mg/kg, 10 µg/kg to 100 µg/kg, 100 µg to 1 mg/kg, and 500 µg/kg to 1 mg/kg. Exemplary treatment regimens and outcomes are summarized in Table 9. Alternative regimens include: (1) 1 x IC₅₀, or 40 µg/kg initial dose and 0.5 x IC₅₀, or 20 µg/kg, 2 weeks later; (2) 10 x IC₅₀ initial dose and 5 x IC₅₀ 2 weeks later, or 100 x IC₅₀ and 50 x IC₅₀ 2 weeks later.

**TABLE 9: Examples of Clinical Cases**

| **Patient No.** | **Status prior to treatment** | **Treatment Regimen** | **Outcome** |
|---|---|---|---|
| **Patient 1** | No clinical signs, postmenopausal and/or over 60 years old | 0.01-1 mg/kg biweekly for 4-24 weeks | Maintenance and/or increase of muscle/bone mass |
| **Patient 2** | Mild clinical signs, muscle wasting and/or bone loss | 0.01-20 mg/kg weekly for 4 more weeks | Maintenance and/or increase of muscle/bone mass |
| **Patient 3** | Advanced stage of osteoporosis | 0.01-20 mg/kg twice weekly for 6 or more weeks | Improvement of clinical signs, maintenance and/or increase of muscle/bone mass |
| **Patient 4** | Severe muscle and bone loss | 0.01-20 mg/kg daily for 6 or more weeks | Improvement of clinical signs, reduction in severity of symptoms and/or increase of muscle/bone mass |

### Example 12: Treatment of Metabolic Disorders

Inhibitors of GDF-8, such as, for example, ActRIIB fusion polypeptides, are useful for treatment of metabolic disorders such as type 2 diabetes, impaired glucose tolerance, metabolic syndrome (*e.g*., syndrome X), insulin resistance induced by trauma (*e.g*., burns or nitrogen imbalance), and adipose tissue disorders (*e.g*., obesity). The ActRIIB fusion polypeptides antibodies may be used to treat a subject at disease onset or having an established metabolic disease.

Efficacy of ActRIIB fusion polypeptides for treatment of metabolic disorders, *e.g.,* type 2 diabetes and/or obesity, is confirmed using well established murine models of obesity, insulin resistance and type 2 diabetes, including ob/ob, db/db, and strains carrying the lethal yellow mutation. Insulin resistance can also be induced by high fat or high caloric feeding of certain strains of mice, including C57BL/6J. Similarly to humans, these rodents develop insulin resistance, hyperinsuliemia, dyslipidemia, and deterioration of glucose homeostasis resulting in hyperglycemia. Outcome assessments are based on serum measurements of glucose, insulin and lipids. Measures of improved insulin sensitivity can be determined by insulin tolerance tests and glucose tolerance tests. More sensitive techniques would include the use of euglycemic-hyperinsulinemic clamps for assessing improvements is glycemic control and insulin sensitivity. In addition, the clamp techniques would allow a quantitative assessment of the role of the major glucose disposing tissues (muscle, adipose, and liver) in improved glycemic control.

In one study, treatment with an ActRIIB fusion polypeptide such one set out in SEQ ID NO:3 (IP injection) or vehicle is conducted for one week to six months. The treatment protocol could vary, with testing of different doses and treatment regimens (*e.g*., daily, weekly, or bi-weekly injections). It is anticipated that mice treated with the fusion polypeptide would have greater glucose uptake, increased glycolysis and glycogen synthesis, lower free fatty acids and triglycerides in the serum as compared to mice receiving placebo treatment.

The ActRIIB fusion polypeptides are also used to prevent and/or to reduce severity and/or the symptoms of the disease. It is anticipated that the ActRIIB fusion polypeptides would be administered as a subcutaneous injection as frequently as once per day and as infrequently as once per month. Treatment duration could range from one month and several years.

To test the clinical efficacy of ActRIIB fusion polypeptides in humans, subjects suffering from or at risk for type 2 diabetes are identified and randomized to a treatment group. Treatment groups include a placebo group and one to three groups receiving ActRIIB fusion polypeptides (different doses). Individuals are followed prospectively for one month to three years to assess changes in glucose metabolism. It is anticipated that individuals receiving treatment would exhibit an improvement.

The ActRIIB fusion polypeptides are administered as the sole active compound or in combination with another compound or composition. When administered as the sole active compound or in combination with another compound or composition, the dosage may be from approximately 1 µg/kg to 20 mg/kg, depending on the severity of the symptoms and the progression of the disease. The appropriate effective dose is selected by a treating clinician from the following ranges: 1 µg/kg to 20 mg/kg, 1 µg/kg to 10 mg/kg, 1 µg/kg to 1 mg/kg, 10 µg/kg to 1 mg/kg, 10 µg/kg to 100 µg/kg, 100 µg to 1 mg/kg, and 500 µg/kg to 1 mg/kg. Exemplary treatment regimens and outcomes are summarized in Table 7.

**TABLE 7: Examples of Clinical Cases**

| **Patient No.** | **Status prior to treatment** | **Treatment Regimen** | **Outcome** |
|---|---|---|---|
| **Patient 1** | No clinical signs, family history of type 2 diabetes | 0.01-1 mg/kg every 4 weeks for 48 weeks | Prevention of type 2 diabetes |
| **Patient 2** | Mild clinical signs of syndrome X | 0.01-20 mg/kg weekly for 4 more weeks | Improved insulin tolerance and glucose metabolism, and lower blood pressure |
| **Patient 3** | Advanced stage of type 2 diabetes | 0.01-20 mg/kg twice weekly for 6 or more weeks | Improvement of clinical signs, reduction in severity of symptoms and/or increase in muscle mass/ body fat ratio |
| **Patient 4** | Severe insulin resistance and/obesity | 0.01-20 mg/kg daily for 6 or more weeks | Improvement of clinical signs, reduction in severity of symptoms and/or decrease in body fat |

## Claims

1. Use of an Activin type IIB receptor (ActRIIB) fusion polypeptide in manufacture of a medicament for treatment or prevention of a bone degenerative disorder in a mammal, wherein the ActRIIB fusion polypeptide comprises (a) an amino acid sequence that is at least 95% identical to amino acids 23 to 138 of SEQ ID NO:3 and is capable of binding to growth and differentiation factor-8 (GDF-8) and (b) the constant region of an IgG, IgA, IgE, or IgM antibody.

2. The use of claim 1, wherein the mammal is human.

3. The use of claim 1, wherein the medicament is to be administered to a mammal in need of treatment or prevention of a disorder chosen from at least one of osteoarthritis and osteoporosis.

4. The use of claim 1, wherein said ActRIIB fusion polypeptide is to be administered at the effective amount chosen from 1 µg/kg to 20 mg/kg, 1 µg/kg to 10 mg/kg, 1 µg/kg to 1 mg/kg, 10 µg/kg to 1 mg/kg, 10 µg/kg. to 100 µg/kg, 100 µg to 1 mg/kg, and 500 µg/kg to 1 mg/kg.

5. The use of claim 1, wherein the first amino acid sequence of said ActRIIB fusion polypeptide comprises amino acids 23 to 138 of SEQ ID NO:3.

6. The use of claim 1, wherein the first amino acid sequence of said ActRIIB fusion polypeptide comprises amino acids 19 to 134 of SEQ ID NO:1.

7. The use of claim 1, wherein the second amino acid sequence of said ActRIIB fusion polypeptide comprises a sequence chosen from (a) the Fc fragment of IgG, (b) the Fc fragment of IgG1, (c) the Fc fragment of IgG4, and (d) amino acids 148 to 378 of SEQ ID NO:3.

8. The use of claim 1, wherein the sequence of the ActRIIB fusion polypeptide is set out in SEQ ID NO:3.

9. The use of claim 1, wherein circulatory half life of the ActRIIB fusion polypeptide exceeds 5 days.

10. The use of claim 1, wherein the fusion protein is encoded by a nucleic acid that hybridizes to the sequence of SEQ ID NO:4 under stringent hybridization conditions, and wherein the nucleic acid encodes a fusion protein that is capable of binding to GDF-8.

11. A fusion protein comprising the amino acid sequence of SEQ ID NO:3.

12. An isolated nucleic acid encoding the fusion protein of claim 11.

13. The nucleic acid of claim 12, wherein said nucleic acid is set out in SEQ ID NO:4.

14. An expression vector, comprising the nucleic acid of claim 12.

15. A host cell comprising the vector of claim 14.

16. A method for identifying inhibitors of GDF 8, comprising:
(a) preparing a first binding mixture comprising the ActRIIB fusion polypeptide of claim 11 and GDF 8;
(b) measuring the amount of binding between the ActRIIB fusion polypeptide and GDF 8 in the first mixture;
(c) preparing a second binding mixture comprising the ActRIIB fusion polypeptide, GDF 8 and a test compound; and
(d) measuring the amount of binding between the ActRIIB fusion polypeptide and GDF 8 in the second mixture.

17. Use of an ActRIIB fusion polypeptide in manufacture of a medicament for increasing trabecular bone density, wherein the ActRIIB fusion polypeptide comprises (a) an amino acid sequence that is at least 95% identical to amino acids 23 to 138 of SEQ ID NO:3 and is capable of binding to growth and differentiation factor-8 (GDF-8) and (b) the constant region of an IgG, IgA, IgE, or IgM antibody.

18. The use of claim 17, wherein ActRIIB fusion polypeptide is to be administered to the mammal at effective dose from 1 µg/kg to 20 mg/kg, 1 µg/kg to 10 mg/kg, 1 µg/kg to 1 mg/kg, 10µg/kg to 1 mg/kg, 10µg/kg to 100µg/kg, 100µg to 1 mg/kg, or 500µg/kg to 1 mg/kg.

19. The use of claim 17, wherein the first amino acid sequence of the ActRIIB fusion polypeptide comprises amino acids 23 to 138 of SEQ ID NO:3.

20. The use of claim 17, wherein the first amino acid sequence of ActRIIB fusion polypeptide comprises amino acids 19 to 134 of SEQ ID NO:1.

21. The use of claim 17, wherein the second amino acid sequence of the ActRIIB fusion polypeptide comprises (a) the Fc fragment of IgG, (b) the Fc fragment of IgG1, (c) the Fc fragment of IgG4, or (d) amino acids 148 to 378 of SEQ ID NO:3.

22. The use of claim 17, wherein the amino acid sequence of the ActRIIB fusion polypeptide is (a) set out in SEQ ID NO:3 or (b) encoded by a nucleic acid that hybridizes to the sequence of SEQ ID NO:4 under stringent hybridization conditions, wherein the nucleic acid encodes a fusion protein that is capable of binding to GDF-8.

23. The use of claim 17, wherein circulatory half life of the ActRIIB fusion polypeptide exceeds 5 days.

## Patentansprüche

1. Verwendung von Acitvin Typ IIB Rezeptor (ActRI1B)-Fusionspolypeptid zur Herstellung eines Medikaments für die Behandlung oder Prävention von degenerativen Störungen der Knochen in Säugern, wobei das ActRIIB-Fusionspolypeptid folgendes umfasst: (a) eine Aminosäuresequenz, welche zu mindestens 95% identisch mit den Aminosäuren 23 bis 138 der SEQ ID NO:3 ist und zur Bindung an den Wachstums- und Differenzierungsfaktor 8 (GDF-8) fähig ist, und (b) die konstante Region von einem IgG, IgA, IgE, oder IgM Antikörper.

2. Die Verwendung nach Anspruch 1, wobei der Säuger menschlich ist.

3. Die Verwendung nach Anspruch 1, wobei das Medikament einem Säuger verabreicht werden soll, welcher eine Behandlung oder Prävention einer Störung, ausgewählt von wenigstens einem aus Osteoarthritis und Osteoporosis, benötigt.

4. Die Verwendung nach Anspruch 1, wobei das ActRI1B-Fusionspolypeptid in einer wirkungsvollen Menge verabreicht werden soll, ausgewählt aus 1 µg/kg bis 20 mg/kg, 1 µg/kg bis 10 mg/kg, 1 µg/kg bis 1 mg/kg, 10 µg/kg bis 1 mg/kg, 10 µg/kg bis 100 µg/kg, 100 µg/kg bis 1 mg/kg und 500 µg/kg bis 1 mg/kg.

5. Die Verwendung nach Anspruch 1, wobei die erste Aminosäuresequenz des ActRIIB-Fusionspolypeptids die Aminosäuren 23 bis 138 der SEQ ID NO:3 umfasst.

6. Die Verwendung nach Anspruch 1, wobei die erste Aminosäuresequenz des ActRIIB-Fusionspolypeptids die Aminosäuren 19 bis 134 der SEQ ID NO:1 umfasst.

7. Die Verwendung nach Anspruch 1, wobei die zweite Aminosäuresequenz des ActRIIB-Fusionspolypeptids eine Sequenz umfasst, welche ausgewählt ist aus (a) das Fc Fragment von IgG, (b) das Fc Fragment von IgG1, (c) das Fc Fragment von IgG4, und (d) die Aminosäuren 148 bis 378 der SEQ ID NO:3.

8. Die Verwendung nach Anspruch 1, wobei die Sequenz des ActRIIB-Fusionspolypeptids in der SEQ ID NO:3 festgelegt ist.

9. Die Verwendung nach Anspruch 1, wobei die Halbwertszeit des ActRIIB-Fusionspolypeptids im Kreislauf 5 Tage überschreitet.

10. Die Verwendung nach Anspruch 1, wobei das Fusionsprotein durch eine Nukleinsäure kodiert ist, welche unter stringenten Hybridisierungsbedingungen mit der Sequenz von SEQ ID NO:4 hybridisiert, und wobei die Nukleinsäure für ein Fusionsprotein kodieren, welches fähig ist, an GDF-8 zu binden.

11. Ein Fusionsprotein umfassend die Aminosäuresequenz von SEQ ID NO:3.

12. Eine isolierte Nukleinsäure, welche für das Fusionsprotein von Anspruch 11 kodiert.

13. Die Nukleinsäure nach Anspruch 12, wobei die Nukleinsäure in der SEQ ID NO:4 festgelegt ist.

14. Ein Expressionsvektor, welcher die Nukleinsäure nach Anspruch 12 umfasst.

15. Eine Wirtszelle, welche den Vektor nach Anspruch 14 umfasst.

16. Ein Verfahren zur Identifizierung von Inhibitoren von GDF, umfassend:
(a) das Aufbereiten einer ersten Bindungs-Mischung umfassend das ActRIIB-Fusionspolypeptid nach Anspruch 11 und GDF-8;
(b) das Messen der Anzahl von Bindungen zwischen dem ActRIIB-Fusionspolypeptid und GDF-8 in der ersten Mischung;
(c) das Aufbereiten einer zweiten Bindungs-Mischung umfassend das ActRIIB-Fusionspolypeptid, GDF-8 und eine Testsubstanz; und
(d) das Messen der Anzahl von Bindungen zwischen dem ActRIIB-Fusionspolypeptid und GDF-8 in der zweiten Mischung.

17. Verwendung eines ActRIIB-Fusionspolypeptid in der Herstellung eines Medikaments zur Erhöhung der trabekulären Knochendichte, wobei das ActRIIB-Fusionspolypeptid folgendes umfasst: (a) eine Aminosäuresequenz, welche zu mindestens 95% identisch mit den Aminosäuren 23 bis 138 der SEQ ID NO:3 ist und zur Bindung an den Wachstums- und Differenzierungsfaktor 8 (GDF-8) fähig ist, und (b) die konstante Region von einem IgG, IgA, IgE, oder IgM Antikörper.

18. Die Verwendung nach Anspruch 17, wobei das ActRIIB-Fusionspolypeptid einem Säuger verabreicht werden soll, in einer wirkungsvollen Menge ausgewählt aus 1 µg/kg bis 20 mg/kg, 1 µg/kg bis 10 mg/kg, 1 µg/kg bis 1 mg/kg, 10 µg/kg bis 1 mg/kg, 10 µg/kg bis 100 µg/kg, 100 µg/kg bis 1 mg/kg und 500 µg/kg bis 1 mg/kg.

19. Die Verwendung nach Anspruch 17, wobei die erste Aminosäuresequenz des ActRIIB-Fusionspolypeptids die Aminosäuren 23 bis 138 der SEQ ID NO:3 umfasst.

20. Die Verwendung nach Anspruch 17, wobei die erste Aminosäuresequenz des ActRIIB-Fusionspolypeptids die Aminosäuren 19 bis 134 der SEQ ID NO:1 umfasst.

21. Die Verwendung nach Anspruch 17, wobei die zweite Aminosäuresequenz des ActRIIB-Fusionspolypeptids (a) das Fc Fragment von IgG, (b) das Fc Fragment von IgG1, (c) das Fc Fragment von IgG4, und/oder (d) die Aminosäuren 148 bis 378 der SEQ ID NO:3 umfasst.

22. Die Verwendung nach Anspruch 17, wobei die Aminosäuresequenz des ActRIIB-Fusionspolypeptids (a) (iss set out) in der SEQ ID NO:3 oder (b) durch eine Nukleinsäure kodiert ist, welche unter stringenten Hybridisierungsbedingungen mit der Sequenz von SEQ ID NO:4 hybridisiert, und wobei die Nukleinsäure für ein Fusionsprotein kodieren, welches fähig ist, an GDF-8 zu binden.

23. Die Verwendung nach Anspruch 17, wobei die Halbwertszeit des ActRIIB-Fusionspolypeptids im Kreislauf 5 Tage überschreitet.

## Revendications

1. Utilisation d'un polypeptide de fusion (ActRIIB) du récepteur de type IIB de l'activine dans la production d'un médicament pour le traitement ou la prévention d'une maladie dégénérative osseuse chez un mammifère, le polypeptide de fusion ActRIIB comprenant (a) une séquence d'acides aminés qui est identique à au moins 95 % aux acides aminés 23 à 138 de SEQ ID N° : 3 et est capable de se lier au facteur 8 de croissance et de différenciation (GDF-8) et (b) la région constante d'un anticorps d'IgG, IgA, IgE ou IgM.

2. Utilisation selon la revendication 1, dans laquelle le mammifère est un être humain.

3. Utilisation selon la revendication 1, dans laquelle le médicament devra être administré à un mammifère qui nécessite le traitement ou la prévention d'un trouble choisi parmi au moins une maladie telle que l'arthrose et l'ostéoporose.

4. Utilisation selon la revendication 1, dans laquelle ledit polypeptide de fusion ActRIIB doit être administré en une quantité efficace choisie parmi 1 µg à 20 mg/kg, 1 µg/kg à 10 mg/kg, 1 µg/kg à 1 mg/kg, 10 µg/kg à 1 mg/kg, 10 µg/kg à 100 µg/kg, 100 µg à 1 mg/kg et 500 µg/kg à 1 mg/kg.

5. Utilisation selon la revendication 1, dans laquelle la première séquence d'acides aminés dudit polypeptide de fusion ActRIIB comprend les acides aminés 23 à 138 de SEQ ID N° : 3.

6. Utilisation selon la revendication 1, dans laquelle la première séquence d'acides aminés dudit polypeptide de fusion ActRIIB comprend les acides aminés 19 à 134 de SEQ ID N° : 1.

7. Utilisation selon la revendication 1, dans laquelle la deuxième séquence d'acides aminés dudit polypeptide de fusion ActRIIB comprend une séquence choisie parmi
(a) le fragment Fc de IgG, (b) le fragment Fc de IgG1, (c) le fragment Fc de IgG4 et (d), les acides aminés 148 à 378 de SEQ ID N° : 3.

8. Utilisation selon la revendication 1, dans laquelle la séquence du polypeptide de fusion ActRIIB est définie dans SEQ ID N° : 3.

9. Utilisation selon la revendication 1, dans laquelle la demi-vie dans la circulation du polypeptide de fusion ActRIIB dépasse 5 jours.

10. Utilisation selon la revendication 1, dans laquelle la protéine de fusion est codée par un acide nucléique qui s'hybride à la séquence de SEQ ID N° : 4 dans des conditions d'hybridation stringentes et dans laquelle l'acide nucléique code pour une protéine de fusion qui est capable de se lier à GDF-8.

11. Protéine de fusion comprenant la séquence d'acides aminés de SEQ ID N° : 3.

12. Acide nucléique isolé codant pour la protéine de fusion selon la revendication 11.

13. Acide nucléique selon la revendication 12, ledit acide nucléique étant défini dans SEQ ID N° : 4.

14. Vecteur d'expression comprenant l'acide nucléique selon la revendication 12.

15. Cellule hôte comprenant le vecteur de la revendication 14.

16. Procédé d'identification d'inhibiteurs de GDF 8, comprenant :
(a) la préparation d'un premier mélange de liaison comprenant le polypeptide de fusion ActRIIB selon la revendication 11 et GDF 8 ;
(b) la mesure de la quantité de liaison entre le polypeptide de fusion ACTRIIB et GDF 8 dans le premier mélange ;
(c) la préparation d'un deuxième mélange de liaison comprenant le polypeptide de fusion ActRIIB, GDF 8 et un composé de test ; et
(d) la mesure de la quantité de liaison entre le polypeptide de fusion ActRIIB et GDF 8 dans le deuxième mélange.

17. Utilisation d'un polypeptide de fusion ActRIIB dans la production d'un médicament destiné à renforcer la densité de l'os trabéculaire, le polypeptide de fusion ActRIIB comprenant (a) une séquence d'acides aminés qui est identique à au moins 95 % aux acides aminés 23 à 138 de SEQ ID N° : 3 et est capable de se lier au facteur 8 de croissance et de différenciation (GDF-8) et (b) la région constante d'un anticorps d'IgG, IgA, IgE ou IgM.

18. Utilisation selon la revendication 17, dans laquelle le polypeptide de fusion ActRIIB doit être administré au mammifère en une quantité efficace choisie parmi 1 µg/kg à 20 mg/kg, 1 µg/kg à 10 mg/kg, 1 µg/kg à 1 mg/kg, 10 µg/kg à 1 mg/kg, 10 µg/kg à 100 µg/kg, 100 µg à 1 mg/kg ou 500 µg/kg à 1 mg/kg.

19. Utilisation selon la revendication 17, dans laquelle la première séquence d'acides aminés du polypeptide de fusion ActRIIB comprend les acides aminés 23 à 138 de SEQ ID N° : 3.

20. Utilisation selon la revendication 17, dans laquelle la première séquence d'acides aminés du polypeptide de fusion ActRIIB comprend les acides aminés 19 à 134 de SEQ ID N° : 1.

21. Utilisation selon la revendication 17, dans laquelle la deuxième séquence d'acides aminés du polypeptide de fusion ActRIIB comprend (a) le fragment Fc de IgG, (b) le fragment Fc de IgG1, (c) le fragment Fc de IgG4 et (d), les acides aminés 148 à 378 de SEQ ID N° : 3.

22. Utilisation selon la revendication 17, dans laquelle la séquence d'acides aminés du polypeptide de fusion ActRIIB est (a) définie dans SEQ ID N° : 3 ou (b) est codée par un acide nucléique qui s'hybride à la séquence de SEQ ID N° : 4 dans des conditions d'hybridation stringentes dans laquelle l'acide nucléique code pour une protéine de fusion qui est capable de se lier à GDF-8.

23. Utilisation selon la revendication 17, dans laquelle la demi-vie dans la circulation du polypeptide de fusion ActRIIB dépasse 5 jours.


### SEQUENCE LISTING

<110> Wyeth
   Wolfman, Neil
   Bouxsein, Mary
<120> ActRIIB Fusion polypeptides and Uses Therefor
<130> 8702.093-304
<160> 6
<170> PatentIn version 3.1
<210> 1
   <211> 512
   <212> PRT
   <213> Human
<400> 1
   Met Thr Ala Pro Trp Val Ala Leu Ala Leu Leu Trp Gly Ser Leu Cys
   1               5                   10                  15
   Ala Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Gln Asp Lys Arg Leu His Cys Tyr Ala Ser Trp Ala
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
   100 105 110
   Phe Thr His Leu Pro Glu Ala Gly Gly Pro Glu Val Thr Tyr Glu Pro
   115 120 125
   Pro Pro Thr Ala Pro Thr Leu Leu Thr Val Leu Ala Tyr Ser Leu Leu
   130 135 140
   Pro Ile Gly Gly Leu Ser Leu Ile Val Leu Leu Ala Phe Trp Met Tyr
   145 150 155 160
   Arg His Arg Lys Pro Pro Tyr Gly His Val Asp Ile His Glu Asp Pro
   165 170 175
   Gly Pro Pro Pro Pro Ser Pro Leu Val Gly Leu Lys Pro Leu Gln Leu
   180 185 190
   Leu Glu Ile Lys Ala Arg Gly Arg Phe Gly Cys Val Trp Lys Ala Gln
   195 200 205
   Leu Met Asn Asp Phe Val Ala Val Lys Ile Phe Pro Leu Gln Asp Lys
   210 215 220
   Gln Ser Trp Gln Ser Glu Arg Glu Ile Phe Ser Thr Pro Gly Met Lys
   225 230 235 240
   His Glu Asn Leu Leu Gln Phe Ile Ala Ala Glu Lys Arg Gly Ser Asn
   245 250 255
   Leu Glu Val Glu Leu Trp Leu Ile Thr Ala Phe His Asp Lys Gly Ser
   260 265 270
   Leu Thr Asp Tyr Leu Lys Gly Asn Ile Ile Thr Trp Asn Glu Leu Cys
   275 280 285
   His Val Ala Glu Thr Met Ser Arg Gly Leu Ser Tyr Leu His Glu Asp
   290 295 300
   Val Pro Trp Cys Arg Gly Glu Gly His Lys Pro Ser Ile Ala His Arg
   305 310 315 320
   Asp Phe Lys Ser Lys Asn Val Leu Leu Lys Ser Asp Leu Thr Ala Val
   325 330 335
   Leu Ala Asp Phe Gly Leu Ala Val Arg Phe Glu Pro Gly Lys Pro Pro
   340 345 350
   Gly Asp Thr His Gly Gln Val Gly Thr Arg Arg Tyr Met Ala Pro Glu
   355 360 365
   Val Leu Glu Gly Ala Ile Asn Phe Gln Arg Asp Ala Phe Leu Arg Ile
   370 375 380
   Asp Met Tyr Ala Met Gly Leu Val Leu Trp Glu Leu Val Ser Arg Cys
   385 390 395 400
   Lys Ala Ala Asp Gly Pro Val Asp Glu Tyr Met Leu Pro Phe Glu Glu
   405 410 415
   Glu Ile Gly Gln His Pro Ser Leu Glu Glu Leu Gln Glu Val Val Val
   420 425 430
   His Lys Lys Met Arg Pro Thr Ile Lys Asp His Trp Leu Lys His Pro
   435 440 445
   Gly Leu Ala Gln Leu Cys Val Thr Ile Glu Glu Cys Trp Asp His Asp
   450 455 460
   Ala Glu Ala Arg Leu Ser Ala Gly Cys Val Glu Glu Arg Val Ser Leu
   465 470 475 480
   Ile Arg Arg Ser Val Asn Gly Thr Thr Ser Asp Cys Leu Val Ser Leu
   485 490 495
   Val Thr Ser Val Thr Asn Val Asp Leu Pro Pro Lys Glu Ser Ser Ile
   500 505 510
<210> 2
   <211> 375
   <212> PRT
   <213> Human
<400> 2
   Met Gln Lys Leu Gln Leu Cys Val Tyr Ile Tyr Leu Phe Met Leu Ile
   1 5 10 15
   Val Ala Gly Pro Val Asp Leu Asn Glu Asn Ser Glu Gln Lys Glu Asn
   20 25 30
   Val Glu Lys Glu Gly Leu Cys Asn Ala Cys Thr Trp Arg Gln Asn Thr
   35 40 45
   Lys Ser Ser Arg Ile Glu Ala Ile Lys Ile Gln Ile Leu Ser Lys Leu
   50 55 60
   Arg Leu Glu Thr Ala Pro Asn Ile Ser Lys Asp Val Ile Arg Gln Leu
   65 70 75 80
   Leu Pro Lys Ala Pro Pro Leu Arg Glu Leu Ile Asp Gln Tyr Asp Val
   85 90 95
   Gln Arg Asp Asp Ser Ser Asp Gly Ser Leu Glu Asp Asp Asp Tyr His
   100 105 110
   Ala Thr Thr Glu Thr Ile Ile Thr Met Pro Thr Glu Ser Asp Phe Leu
   115 120 125
   Met Gln Val Asp Gly Lys Pro Lys Cys Cys Phe Phe Lys Phe Ser Ser
   130 135 140
   Lys Ile Gln Tyr Asn Lys Val Val Lys Ala Gln Leu Trp Ile Tyr Leu
   145 150 155 160
   Arg Pro Val Glu Thr Pro Thr Thr Val Phe Val Gln Ile Leu Arg Leu
   165 170 175
   Ile Lys Pro Met Lys Asp Gly Thr Arg Tyr Thr Gly Ile Arg Ser Leu
   180 185 190
   Lys Leu Asp Met Asn Pro Gly Thr Gly Ile Trp Gln Ser Ile Asp Val
   195 200 205
   Lys Thr Val Leu Gln Asn Trp Leu Lys Gln Pro Glu Ser Asn Leu Gly
   210 215 220
   Ile Glu Ile Lys Ala Leu Asp Glu Asn Gly His Asp Leu Ala Val Thr
   225 230 235 240
   Phe Pro Gly Pro Gly Glu Asp Gly Leu Asn Pro Phe Leu Glu Val Lys
   245 250 255
   Val Thr Asp Thr Pro Lys Arg Ser Arg Arg Asp Phe Gly Leu Asp Cys
   260 265 270
   Asp Glu His Ser Thr Glu Ser Arg Cys Cys Arg Tyr Pro Leu Thr Val
   275 280 285
   Asp Phe Glu Ala Phe Gly Trp Asp Trp Ile Ile Ala Pro Lys Arg Tyr
   290 295 300
   Lys Ala Asn Tyr Cys Ser Gly Glu Cys Glu Phe Val Phe Leu Gln Lys
   305 310 315 320
   Tyr Pro His Thr His Leu Val His Gln Ala Asn Pro Arg Gly Ser Ala
   325 330 335
   Gly Pro Cys Cys Thr Pro Thr Lys Met Ser Pro Ile Asn Met Leu Tyr
   340 345 350
   Phe Asn Gly Lys Glu Gln Ile Ile Tyr Gly Lys Ile Pro Ala Met Val
   355 360 365
   Val Asp Arg Cys Gly Cys Ser
   370 375
<210> 3
   <211> 378
   <212> PRT
   <213> Chimera
<400> 3
   Met Lys Phe Leu Val Asn Val Ala Leu Val Phe Met Val Val Tyr Ile
   1 5 10 15
   Ser Tyr Ile Tyr Ala Thr Ser Gly Arg Gly Glu Ala Glu Thr Arg Glu
   20 25 30
   Cys Ile Tyr Tyr Asn Ala Asn Trp Glu Leu Glu Arg Thr Asn Gln Ser
   35 40 45
   Gly Leu Glu Arg Cys Glu Gly Glu Gln Asp Lys Arg Leu His Cys Tyr
   50 55 60
   Ala Ser Trp Arg Asn Ser Ser Gly Thr Ile Glu Leu Val Lys Lys Gly
   65 70 75 80
   Cys Trp Leu Asp Asp Phe Asn Cys Tyr Asp Arg Gln Glu Cys Val Ala
   85 90 95
   Thr Glu Glu Asn Pro Gln Val Tyr Phe Cys Cys Cys Glu Gly Asn Phe
   100 105 110
   Cys Asn Glu Arg Phe Thr His Leu Pro Glu Ala Gly Gly Pro Glu Val
           115                 120                 125
   Thr Tyr Glu Pro Pro Pro Thr Ala Pro Thr Gly Gly Arg Gly Asp Asp
       130                 135                 140
   Asp Asp Lys Thr Arg Ser Arg Asp Lys Thr His Thr Cys Pro Pro Cys
   145                 150                 155                 160
   Pro Ala Pro Glu Leu Leu Gly Gly Pro Ser Val Phe Leu Phe Pro Pro
                   165                 170                 175
   Lys Pro Lys Asp Thr Leu Met Ile Ser Arg Thr Pro Glu Val Thr Cys
               180                 185                 190
   Val Val Val Asp Val Ser His Glu Asp Pro Glu Val Lys Phe Asn Trp
           195                 200                 205
   Tyr Val Asp Gly Val Glu Val His Asn Ala Lys Thr Lys Pro Arg Glu
       210                 215                 220
   Glu Gln Tyr Asn Ser Thr Tyr Arg Val Val Ser Val Leu Thr Val Leu
   225                 230                 235                 240
   His Gln Asp Trp Leu Asn Gly Lys Glu Tyr Lys Cys Lys Val Ser Asn
                   245                 250                 255
   Lys Ala Leu Pro Val Pro Ile Glu Lys Thr Ile Ser Lys Ala Lys Gly
               260                 265                 270
   Gln Pro Arg Glu Pro Gln Val Tyr Thr Leu Pro Pro Ser Arg Glu Glu
           275                 280                 285
   Met Thr Lys Asn Gln Val Ser Leu Thr Cys Leu Val Lys Gly Phe Tyr
       290                 295                 300
   Pro Ser Asp Ile Ala Val Glu Trp Glu Ser Asn Gly Gln Pro Glu Asn
   305                 310                 315                 320
   Asn Tyr Lys Thr Thr Pro Pro Val Leu Asp Ser Asp Gly Ser Phe Phe
                   325                 330                 335
   Leu Tyr Ser Lys Leu Thr Val Asp Lys Ser Arg Trp Gln Gln Gly Asn
               340                 345                 350
   Val Phe Ser Cys Ser Val Met His Glu Ala Leu His Asn His Tyr Thr
           355                 360                 365
   Gln Lys Ser Leu Ser Leu Ser Pro Pro Lys
   370 375
<210> 4
   <211> 1134
   <212> DNA
   <213> Chimera
<400> 4
   atgaaattct tagtcaacgt tgcccttgtt tttatggtcg tgtacatttc ttacatctat     60
   gcgactagtg ggcgtgggga ggctgagaca cgggagtgca tctactacaa cgccaactgg    120
   gagctggagc gcaccaacca gagcggcctg gagcgctgcg aaggcgagca ggacaagcgg    180
   ctgcactgct acgcctcctg gcgcaacagc tctggcacca tcgagctcgt gaagaagggc    240
   tgctggctag atgacttcaa ctgctacgat aggcaggagt gtgtggccac tgaggagaac    300
   ccccaggtgt acttctgctg ctgtgaaggc aacttctgca acgagcgctt cactcatttg    360
   ccagaggctg ggggcccgga agtcacgtac gagccacccc cgacagcccc caccggcggc    420
   cgcggagacg acgacgacaa gacgcgttct agagacaaaa ctcacacatg cccaccgtgc    480
   ccagcacctg aactcctggg gggaccgtca gtcttcctct tccccccaaa acccaaggac    540
   accctcatga tctcccggac ccctgaggtc acatgcgtgg tggtggacgt gagccacgaa    600
   gaccctgagg tcaagttcaa ctggtacgtg gacggcgtgg aggtgcataa tgccaagaca    660
   aagccgcggg aggagcagta caacagcacg taccgtgtgg tcagcgtcct caccgtcctg    720
   caccaggact ggctgaatgg caaggagtac aagtgcaagg tctccaacaa agccctccca    780
   gtccccatcg agaaaaccat ctccaaagcc aaagggcagc cccgagaacc acaggtgtac    840
   accctgcccc catcccggga ggagatgacc aagaaccagg tcagcctgac ctgcctggtc    900
   aaaggcttct atcccagcga catcgccgtg gagtgggaga gcaatgggca gccggagaac    960
   aactacaaga ccacgcctcc cgtgctggac tccgacggct ccttcttcct ctatagcaag   1020
   ctcaccgtgg acaagagcag gtggcagcag gggaacgtct tctcatgctc cgtgatgcat   1080
   gaggctctgc acaaccacta cacgcagaag agcctctccc tgtccccgcc taaa         1134
<210> 5
   <211> 4
   <212> PRT
   <213> Artificial
<400> 5
   Gly Ser Gly Ser
   1
<210> 6
   <211> 4
   <212> PRT
   <213> Artificial
<400> 6
   Asp Asp Asp Lys
   1

### SEQUENCE LISTING

<110> Wyeth
   Wolfman, Neil
   Bouxsein, Mary
<120> ActRIIB Fusion polypeptides and Uses Therefor
<130> 8702.093-304
<160> 6
<170> PatentIn version 3.1
<210> 1
   <211> 512
   <212> PRT
   <213> Human
<400> 1
   Met Thr Ala Pro Trp Val Ala Leu Ala Leu Leu Trp Gly Ser Leu Cys
   1               5                   10                  15
   Ala Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Gln Asp Lys Arg Leu His Cys Tyr Ala Ser Trp Ala
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
               100                 105                 110
   Phe Thr His Leu Pro Glu Ala Gly Gly Pro Glu Val Thr Tyr Glu Pro
           115                 120                 125
   Pro Pro Thr Ala Pro Thr Leu Leu Thr Val Leu Ala Tyr Ser Leu Leu
       130                 135                 140
   Pro Ile Gly Gly Leu Ser Leu Ile Val Leu Leu Ala Phe Trp Met Tyr
   145                 150                 155                 160
   Arg His Arg Lys Pro Pro Tyr Gly His Val Asp Ile His Glu Asp Pro
                   165                 170                 175
   Gly Pro Pro Pro Pro Ser Pro Leu Val Gly Leu Lys Pro Leu Gln Leu
               180                 185                 190
   Leu Glu Ile Lys Ala Arg Gly Arg Phe Gly Cys Val Trp Lys Ala Gln
           195                 200                 205
   Leu Met Asn Asp Phe Val Ala Val Lys Ile Phe Pro Leu Gln Asp Lys
       210                 215                 220
   Gln Ser Trp Gln Ser Glu Arg Glu Ile Phe Ser Thr Pro Gly Met Lys
   225                 230                 235                 240
   His Glu Asn Leu Leu Gln Phe Ile Ala Ala Glu Lys Arg Gly Ser Asn
                   245                 250                 255
   Leu Glu Val Glu Leu Trp Leu Ile Thr Ala Phe His Asp Lys Gly Ser
               260                 265                 270
   Leu Thr Asp Tyr Leu Lys Gly Asn Ile Ile Thr Trp Asn Glu Leu Cys
           275                 280                 285
   His Val Ala Glu Thr Met Ser Arg Gly Leu Ser Tyr Leu His Glu Asp
       290                 295                 300
   Val Pro Trp Cys Arg Gly Glu Gly His Lys Pro Ser Ile Ala His Arg
   305                 310                 315                 320
   Asp Phe Lys Ser Lys Asn Val Leu Leu Lys Ser Asp Leu Thr Ala Val
                   325                 330                 335
   Leu Ala Asp Phe Gly Leu Ala Val Arg Phe Glu Pro Gly Lys Pro Pro
               340                 345                 350
   Gly Asp Thr His Gly Gln Val Gly Thr Arg Arg Tyr Met Ala Pro Glu
           355                 360                 365
   Val Leu Glu Gly Ala Ile Asn Phe Gln Arg Asp Ala Phe Leu Arg Ile
       370                 375                 380
   Asp Met Tyr Ala Met Gly Leu Val Leu Trp Glu Leu Val Ser Arg Cys
   385                 390                 395                 400
   Lys Ala Ala Asp Gly Pro Val Asp Glu Tyr Met Leu Pro Phe Glu Glu
                   405                 410                 415
   Glu Ile Gly Gln His Pro Ser Leu Glu Glu Leu Gln Glu Val Val Val
               420                 425                 430
   His Lys Lys Met Arg Pro Thr Ile Lys Asp His Trp Leu Lys His Pro
           435                 440                 445
   Gly Leu Ala Gln Leu Cys Val Thr Ile Glu Glu Cys Trp Asp His Asp
       450                 455                 460
   Ala Glu Ala Arg Leu Ser Ala Gly Cys Val Glu Glu Arg Val Ser Leu
   465                 470                 475                 480
   Ile Arg Arg Ser Val Asn Gly Thr Thr Ser Asp Cys Leu Val Ser Leu
                   485                 490                 495
   Val Thr Ser Val Thr Asn Val Asp Leu Pro Pro Lys Glu Ser Ser Ile
               500                 505                 510
<210> 2
   <211> 375
   <212> PRT
   <213> Human
<400> 2
   Met Gln Lys Leu Gln Leu Cys Val Tyr Ile Tyr Leu Phe Met Leu Ile
   1               5                   10                  15
   Val Ala Gly Pro Val Asp Leu Asn Glu Asn Ser Glu Gln Lys Glu Asn
               20                  25                  30
   Val Glu Lys Glu Gly Leu Cys Asn Ala Cys Thr Trp Arg Gln Asn Thr
           35                  40                  45
   Lys Ser Ser Arg Ile Glu Ala Ile Lys Ile Gln Ile Leu Ser Lys Leu
       50                  55                  60
   Arg Leu Glu Thr Ala Pro Asn Ile Ser Lys Asp Val Ile Arg Gln Leu
   65                  70                  75                  80
   Leu Pro Lys Ala Pro Pro Leu Arg Glu Leu Ile Asp Gln Tyr Asp Val
                   85                  90                  95
   Gln Arg Asp Asp Ser Ser Asp Gly Ser Leu Glu Asp Asp Asp Tyr His
               100                 105                 110
   Ala Thr Thr Glu Thr Ile Ile Thr Met Pro Thr Glu Ser Asp Phe Leu
           115                 120                 125
   Met Gln Val Asp Gly Lys Pro Lys Cys Cys Phe Phe Lys Phe Ser Ser
       130                 135                 140
   Lys Ile Gln Tyr Asn Lys Val Val Lys Ala Gln Leu Trp Ile Tyr Leu
   145                 150                 155                 160
   Arg Pro Val Glu Thr Pro Thr Thr Val Phe Val Gln Ile Leu Arg Leu
                   165                 170                 175
   Ile Lys Pro Met Lys Asp Gly Thr Arg Tyr Thr Gly Ile Arg Ser Leu
               180                 185                 190
   Lys Leu Asp Met Asn Pro Gly Thr Gly Ile Trp Gln Ser Ile Asp Val
           195                 200                 205
   Lys Thr Val Leu Gln Asn Trp Leu Lys Gln Pro Glu Ser Asn Leu Gly
       210                 215                 220
   Ile Glu Ile Lys Ala Leu Asp Glu Asn Gly His Asp Leu Ala Val Thr
   225                 230                 235                 240
   Phe Pro Gly Pro Gly Glu Asp Gly Leu Asn Pro Phe Leu Glu Val Lys
                   245                 250                 255
   Val Thr Asp Thr Pro Lys Arg Ser Arg Arg Asp Phe Gly Leu Asp Cys
               260                 265                 270
   Asp Glu His Ser Thr Glu Ser Arg Cys Cys Arg Tyr Pro Leu Thr Val
           275                 280                 285
   Asp Phe Glu Ala Phe Gly Trp Asp Trp Ile Ile Ala Pro Lys Arg Tyr
       290                 295                 300
   Lys Ala Asn Tyr Cys Ser Gly Glu Cys Glu Phe Val Phe Leu Gln Lys
   305                 310                 315                 320
   Tyr Pro His Thr His Leu Val His Gln Ala Asn Pro Arg Gly Ser Ala
                   325                 330                 335
   Gly Pro Cys Cys Thr Pro Thr Lys Met Ser Pro Ile Asn Met Leu Tyr
               340                 345                 350
   Phe Asn Gly Lys Glu Gln Ile Ile Tyr Gly Lys Ile Pro Ala Met Val
           355                 360                 365
   Val Asp Arg Cys Gly Cys Ser
       370                 375
<210> 3
   <211> 378
   <212> PRT
   <213> Chimera
<400> 3
   Met Lys Phe Leu Val Asn Val Ala Leu Val Phe Met Val Val Tyr Ile
   1               5                   10                  15
   Ser Tyr Ile Tyr Ala Thr Ser Gly Arg Gly Glu Ala Glu Thr Arg Glu
               20                  25                  30
   Cys Ile Tyr Tyr Asn Ala Asn Trp Glu Leu Glu Arg Thr Asn Gln Ser
           35                  40                  45
   Gly Leu Glu Arg Cys Glu Gly Glu Gln Asp Lys Arg Leu His Cys Tyr
       50                  55                  60
   Ala Ser Trp Arg Asn Ser Ser Gly Thr Ile Glu Leu Val Lys Lys Gly
   65                  70                  75                  80
   Cys Trp Leu Asp Asp Phe Asn Cys Tyr Asp Arg Gln Glu Cys Val Ala
   85 90 95
   Thr Glu Glu Asn Pro Gln Val Tyr Phe Cys Cys Cys Glu Gly Asn Phe
   100 105 110
   Cys Asn Glu Arg Phe Thr His Leu Pro Glu Ala Gly Gly Pro Glu Val
   115 120 125
   Thr Tyr Glu Pro Pro Pro Thr Ala Pro Thr Gly Gly Arg Gly Asp Asp
   130 135 140
   Asp Asp Lys Thr Arg Ser Arg Asp Lys Thr His Thr Cys Pro Pro Cys
   145 150 155 160
   Pro Ala Pro Glu Leu Leu Gly Gly Pro Ser Val Phe Leu Phe Pro Pro
   165 170 175
   Lys Pro Lys Asp Thr Leu Met Ile Ser Arg Thr Pro Glu Val Thr Cys
   180 185 190
   Val Val Val Asp Val Ser His Glu Asp Pro Glu Val Lys Phe Asn Trp
   195 200 205
   Tyr Val Asp Gly Val Glu Val His Asn Ala Lys Thr Lys Pro Arg Glu
   210 215 220
   Glu Gln Tyr Asn Ser Thr Tyr Arg Val Val Ser Val Leu Thr Val Leu
   225 230 235 240
   His Gln Asp Trp Leu Asn Gly Lys Glu Tyr Lys Cys Lys Val Ser Asn
   245 250 255
   Lys Ala Leu Pro Val Pro Ile Glu Lys Thr Ile Ser Lys Ala Lys Gly
   260 265 270
   Gln Pro Arg Glu Pro Gln Val Tyr Thr Leu Pro Pro Ser Arg Glu Glu
   275 280 285
   Met Thr Lys Asn Gln Val Ser Leu Thr Cys Leu Val Lys Gly Phe Tyr
   290 295 300
   Pro Ser Asp Ile Ala Val Glu Trp Glu Ser Asn Gly Gln Pro Glu Asn
   305 310 315 320
   Asn Tyr Lys Thr Thr Pro Pro Val Leu Asp Ser Asp Gly Ser Phe Phe
                   325                 330                 335
   Leu Tyr Ser Lys Leu Thr Val Asp Lys Ser Arg Trp Gln Gln Gly Asn
               340                 345                 350
   Val Phe Ser Cys Ser Val Met His Glu Ala Leu His Asn His Tyr Thr
           355                 360                 365
   Gln Lys Ser Leu Ser Leu Ser Pro Pro Lys
       370                 375
<210> 4
   <211> 1134
   <212> DNA
   <213> Chimera
<400> 4
   atgaaattct tagtcaacgt tgcccttgtt tttatggtcg tgtacatttc ttacatctat 60
   gcgactagtg ggcgtgggga ggctgagaca cgggagtgca tctactacaa cgccaactgg 120
   gagctggagc gcaccaacca gagcggcctg gagcgctgcg aaggcgagca ggacaagcgg 180
   ctgcactgct acgcctcctg gcgcaacagc tctggcacca tcgagctcgt gaagaagggc 240
   tgctggctag atgacttcaa ctgctacgat aggcaggagt gtgtggccac tgaggagaac 300
   ccccaggtgt acttctgctg ctgtgaaggc aacttctgca acgagcgctt cactcatttg 360
   ccagaggctg ggggcccgga agtcacgtac gagccacccc cgacagcccc caccggcggc 420
   cgcggagacg acgacgacaa gacgcgttct agagacaaaa ctcacacatg cccaccgtgc 480
   ccagcacctg aactcctggg gggaccgtca gtcttcctct tccccccaaa acccaaggac 540
   accctcatga tctcccggac ccctgaggtc acatgcgtgg tggtggacgt gagccacgaa 600
   gaccctgagg tcaagttcaa ctggtacgtg gacggcgtgg aggtgcataa tgccaagaca 660
   aagccgcggg aggagcagta caacagcacg taccgtgtgg tcagcgtcct caccgtcctg 720
   caccaggact ggctgaatgg caaggagtac aagtgcaagg tctccaacaa agccctccca 780
   gtccccatcg agaaaaccat ctccaaagcc aaagggcagc cccgagaacc acaggtgtac 840
   accctgcccc catcccggga ggagatgacc aagaaccagg tcagcctgac ctgcctggtc 900
   aaaggcttct atcccagcga catcgccgtg gagtgggaga gcaatgggca gccggagaac 960
   aactacaaga ccacgcctcc cgtgctggac tccgacggct ccttcttcct ctatagcaag 1020
   ctcaccgtgg acaagagcag gtggcagcag gggaacgtct tctcatgctc cgtgatgcat 1080
   gaggctctgc acaaccacta cacgcagaag agcctctccc tgtccccgcc taaa 1134
<210> 5
   <211> 4
   <212> PRT
   <213> Artificial
<400> 5
   Gly Ser Gly Ser
   1
<210> 6
   <211> 4
   <212> PRT
   <213> Artificial
<400> 6
   Asp Asp Asp Lys
   1